# EUROPEAN PATENT APPLICATION

(11) **EP 4 838 221 A1**
(43) Date of publication of application: **11.11.2026**
(21) Application number: 25175499.0
(22) Date of filing: 09.05.2025
(51) Int. Cl.: G01N 33/50, C12N 5/09, G01N 33/574

(54) **METHOD AND KIT FOR FUNCTIONAL EX VIVO TESTING OF AN AGENT IN A TUMOUR SAMPLE**

(71) Applicant: FRAUNHOFER-GESELLSCHAFT ZUR FÖRDERUNG DER ANGEWANDTEN FORSCHUNG E.V., 80686 München (DE)
(72) Inventor: WÖHRL, Lukas, 30625 Hannover (DE); WERNO, Christian, 30625 Hannover (DE)
(74) Representative: V.O.

(57) **Abstract**

The present invention refers to a method for testing the effect of an agent, in particular a therapeutic agent, on a tumour sample without cultivation or expansion of the tumour cells of the sample prior to mechanical treatment and/or enzymatic digestion of the tumour sample, wherein a cell type-specific therapeutic effect of the agent is determined. A tumour-specific cytotoxic or cytostatic effect is calculated based on the specific reduction of the number of viable tumour cells in relation to the number of viable immune cells and/or an immune modulatory effect of the agent is determined based on the interaction of immune cells with tumour cells and/or determination of cytokine expression. The invention further relates to a kit for testing the agent.

## Description

The present invention refers to a method for testing the effect of an agent, in particular a therapeutic agent, on a tumour sample without cultivation or expansion of the tumour cells of the sample prior to mechanical treatment and/or enzymatic digestion of the tumour sample, wherein a cell type-specific therapeutic effect of the agent is determined. A tumour-specific cytotoxic or cytostatic effect is calculated based on the specific reduction of the number of viable tumour cells in relation to the number of viable immune cells and/or an immune modulatory effect of the agent is determined based on the interaction of immune cells with tumour cells and/or determination of cytokine expression. The invention further relates to a kit for testing the agent.

### Technical background

Numerous methods for the *ex vivo* testing of agents, in particular therapeutic agents such as immunotherapies or chemotherapies are known. Most of these methods are directed to the testing of immunotherapies or chemotherapies for the treatment of specific tumour types or metastases. A large number of these methods focuses on the expression of tumour-specific markers or marker combinations for testing the therapeutic effect of an immunotherapy or a chemotherapy.

The development of new drugs, in particular cancer drugs, and the prediction of their efficacy in patients are major challenges in modern medicine. Existing preclinical models are intended to enable the testing of therapeutic agents both *in vitro* and *in vivo,* but have considerable disadvantages. *In vitro* models for the testing of therapies are for example cell lines, spheroids, and organoids. These models for testing of tumour therapeutic agents are generated by the expansion of tumour cells, which is very time-consuming, cost-intensive and inefficient. Mäkelä, R. et al., for example describe the testing of therapeutic agents on needle biopsy (Neoplasia (New York, N.Y.), 22(9), 390-398). These models do not provide a cellular microenvironment of a tumour or metastasis, which is the reason that they are only suitable for testing immunotherapies or antibody-drug conjugates (ADCs) to a very limited extent. These *in vitro* methods are only suitable with strong limitations for preclinical drug studies and functional testing of therapeutic agents for cancer patients.

*In vivo* models (PDX) allow the testing of therapeutic agents in the organism, but are very limited regarding desired high throughput, i.e., the parallel testing of several therapeutic agents or lead compounds and are also very time-consuming. Without humanised mice, which are very time-consuming and cost-intensive to generate and maintain, no immunomodulatory effects can be measured, and current regulatory guidelines make animal testing considerably more difficult.

Existing *in vitro* and *in vivo* models are largely based on primary tumours, but new therapeutic agents are particularly needed for metastatic patients, as 90% of cancer patients die from metastases, not from the primary tumour.

So far, a reliable method for testing the effect of an immunotherapy or a chemotherapy in an unspecified tumour type without first isolating or cultivating the tumour cells is missing.

The present invention provides such method which determines the effect of a therapeutic agent through the combined evaluation of the viability of a tumour cell in comparison to a cell in the microenvironment for example via an automated immunofluorescence and image analysis. The results of the viability analysis, cell-cell contacts and cytokine secretion determine the effect of immunotherapy or chemotherapy on a tumour such as a primary tumour or a metastatic tumour.

Based on the method of the present invention therapeutic agents are functionally tested in very small quantities of viable sample material such as biopsies of metastases and/or primary tumours. Advantageously, the tumour cells of the sample are neither enriched nor expanded prior to the testing which allows an accelerated testing of therapeutic agents. In addition, the samples contain the native cellular microenvironment of the tumour, which enables the testing of immunomodulatory therapeutic agents.

### Summary

The present invention refers to a method for testing the effect of an agent on a tumour sample without cultivation or expansion of the tumour sample prior to the following steps of:
a) mechanical treatment and/or enzymatic digestion of the tumour sample to produce a single cell suspension,
b) determining viability of the cells of the single cell suspension and seeding the single cell suspension on a test vessel for 15 to 60 min at room temperature for cell settling in the test vessel,
c) adding the agent to the settling and/or settled cells and incubating the cells on the test vessel with the agent at a single concentration or varying concentrations at 25-40 °C for 1 to 10 days,
d) collecting a sample of the supernatant from the test vessel for determining cytokine expression using a cytokine marker,
e) staining dying and/or dead cells in the test vessel using a marker for dying and/or dead cells,
f) fixing cells in the test vessel for performing immune fluorescence to determine a tumour marker, a marker for an immune cell and a marker for the cell nucleus such as 4', 6-diamin-2-phenylindol (DAPI) or 2'-[4-ethoxyphenyl]-5-[4-methyl-1-piperazinyl]-2,5'-bi-1H-benzimidazole trihydrochloride trihydrate (Hoechst 33342),
g) determining the number of viable immune and/or tumour cells in the test vessel during and/or after incubation, and/or optionally determining a marker for a therapeutic target and/or diagnostic molecule of viable immune and/or tumour cells or all cells of the tumour sample in the test vessel during and/or after incubation,
h) calculating a tumour-specific cytotoxic effect of the agent based on the specific reduction of the number of viable tumour cells in relation to the number of viable immune cells and/or
i) determining an immune modulatory effect of the agent based on the interaction of immune cells with tumour cells and/or optionally determining the cytokine expression. The single cell suspension is for example seeded in the test vessel at a cell density of 123.5 cells per mm² to 2469.1 cells per mm². Optionally, dead cells are removed if the cell viability is below 70 % in step b). The tumour sample is a sample of a primary tumour and/or of a metastasis.

The tumour sample is for example a fresh tumour sample or a fresh biopsy such as a needle biopsy, a fine needle biospy or a liquid biopsy such as a pleural effusion, a pericardial effusion or ascites, or wherein the tumour sample is a frozen liquid biopsy.

Different markers are used. The marker for dying and/or dead cells is for example selected from the group consisting of an antibody, a fluorescent dye, a radioactive dye and a combination thereof. The tumour marker is for example selected from the group consisting of EpCAM (synonymous to CD326), CK1, CK2, CK4, CK5, CK6, CK7, CK8, CK10, CK13, CK14, CK17, CK18, CK19, CK20, E-Cadherin, EMA, TTF-1, Napsin A, p63, p40, p53, GATA3, Mammaglobin, GCDFP-15, CDX2, SATB2, PAX8, Uroplakin II, Uroplakin III, PSA, NKX3.1, Prostein, Claudin-4, Ki-67, CD56, MCSP, gp100 and a combination thereof. The marker for the immune cell is for example selected from the group consisting of CD45, CD3, CD4, CD8, CD19, CD20, CD45, CD14, CD16, CD56, CD25, CD127, CD11b, CD11c, CD15, CD33, CD34, CD38, CD68, CD45RA, CD45RO, HLA-DR, CD66b, CD27, CD28, CD62L, CCR7, CXCR3, CXCR5, FOXP3, CD68 and a combination thereof. For example the tumour marker is EpCAM and the immune cell marker is CD45.

The tumour sample is for example mechanically minced into pieces of 0.5 to 5 mm³ in a suspension medium, incubated for 10 to 60 min at 20 °C to 40 °C and resuspended every 10 min to form the single cell suspension in step a). Alternatively, the tumour sample is a liquid biopsy which is centrifuged in step b), optionally red blood cells are lysed, and the centrifuged cells are resuspended as single cell suspension, for example wherein the cell concentration in the single cell suspension is 0.01 to 1x10⁶ cells/ml. In addition or alternatively, the tumour sample or the single cell suspension of the mechanical treatment is enzymatically digested.

A fluorescence signal of the marker is for example sequentially measured using fluorescence microscopy in channels wherein laser and bandpass filter sets are set to non-overlapping channels.

The agent is for example a therapeutic agent, e.g., selected from the group consisting of a small molecule, a biopharmaceutical such as an antibody, an antibody-drug conjugate (ADC), an oligonucleotide, an oncolytic virus, a Chimeric Antigen Receptor (CAR)-T cell, a CAR-NK cell, a CAR-macrophage cell, a Tumour-Infiltrating Lymphocyte (TIL), and a combination thereof.

The present invention further refers to a kit for testing the effect of an agent on a tumour cell according to a method of the present invention, wherein the kit comprises a vessel, a tumour marker, a marker for an immune cell, and optionally a marker for dying and/or dead cells, a marker for cytokines, and/or a marker for a therapeutic target and/or diagnostic molecule.

### Figures

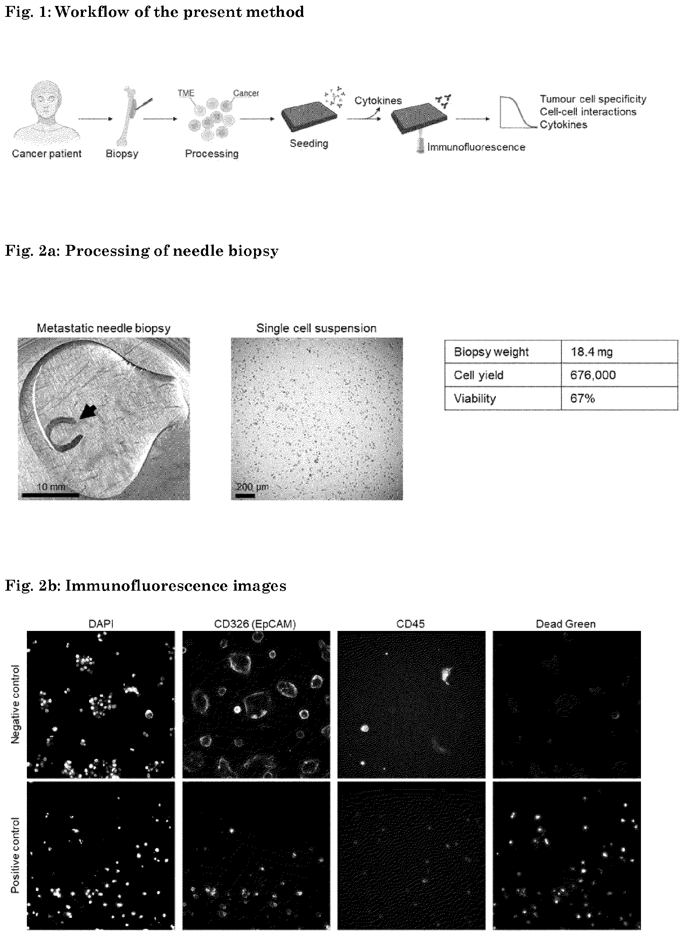

**Fig. 1** shows a schematic representation of the technical workflow of the method of the present invention.

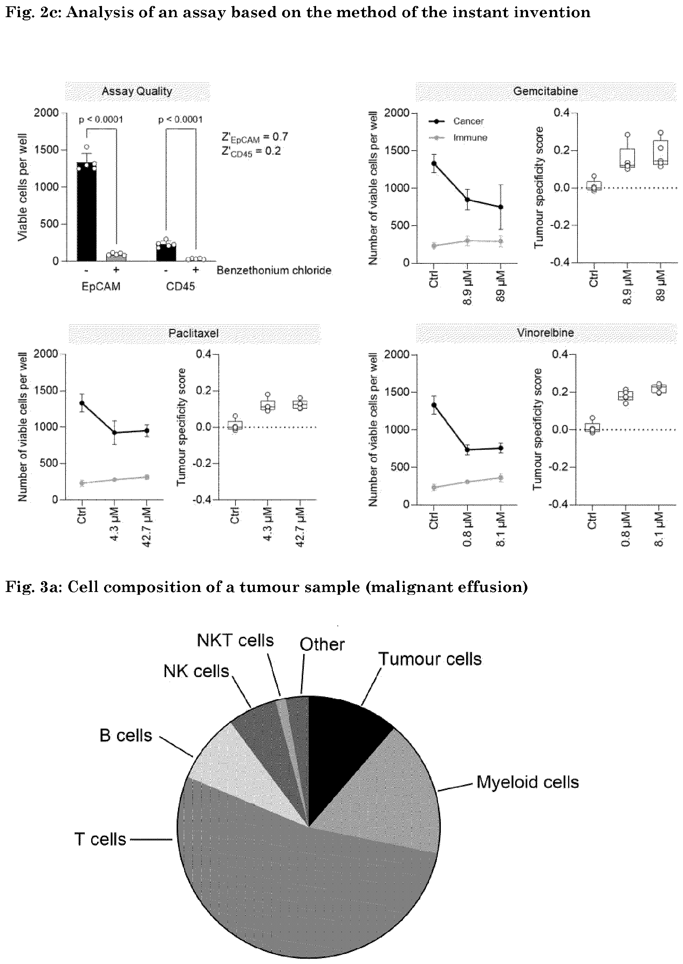

**Fig. 2a to 2c** depicts *ex vivo* drug testing in a needle biopsy from CUP liver metastasis. **Fig. 2a** shows a representative image of a needle biopsy from a cancer of unknown primary (CUP) liver metastasis, along with the biopsy-derived single-cell suspension following mechanical and enzymatic digestion. The accompanying table provides details on the biopsy characteristics before and after processing. **Fig. 2b** depicts representative immunofluorescence images after five days of incubation with therapeutic agents. The images represent one of 16 fields acquired per well of a 384-well plate. The negative control is the solvent (DMSO), while the positive control contains benzethonium chloride. **Fig. 2c** shows an analysis of assay quality and the responsiveness of cancer and immune cells from the needle biopsy to three representative chemotherapeutic agents. The tumor specificity of the therapeutic agents was determined using the formula outlined in the present invention.

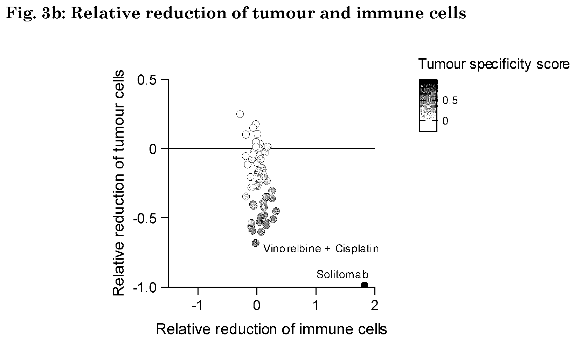

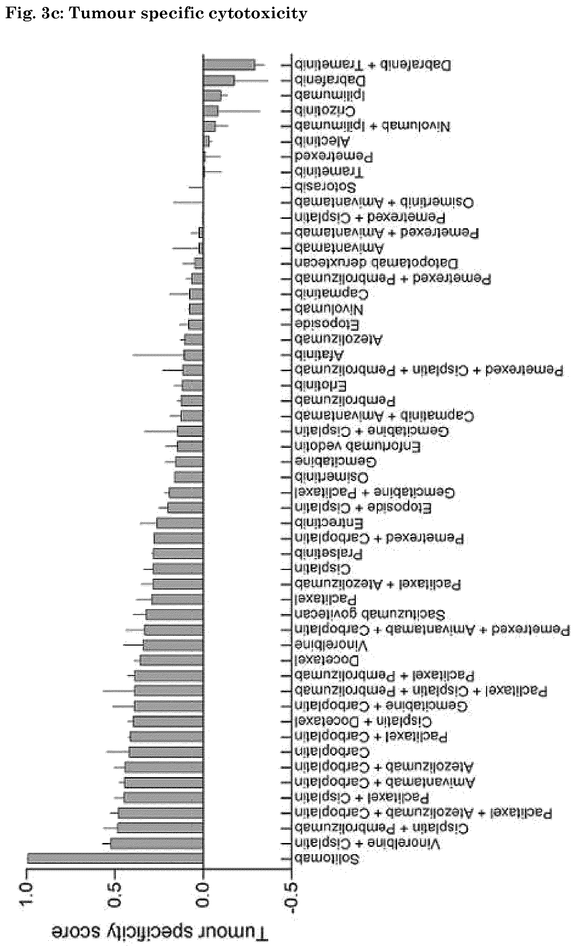

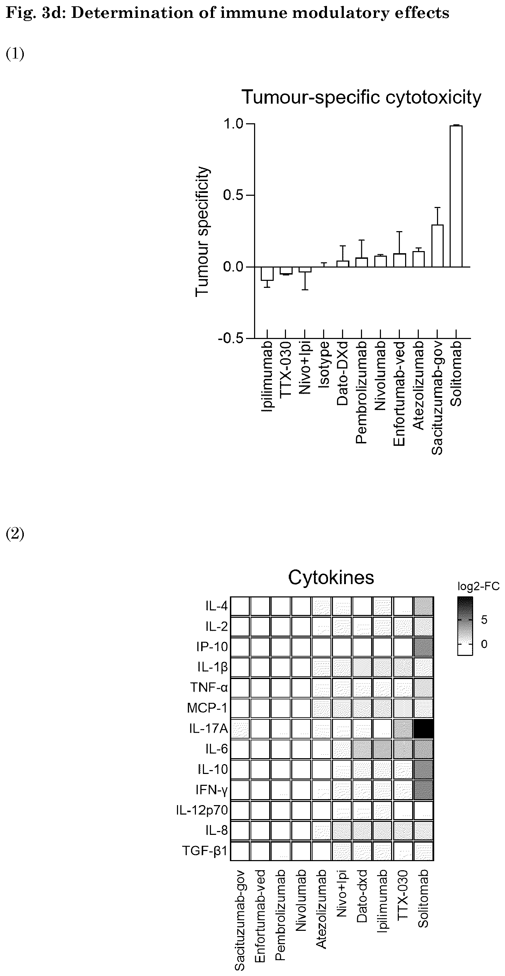

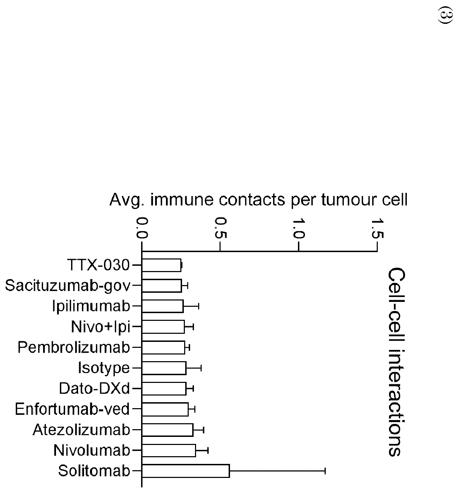

**Fig. 3a to 3d** shows *ex vivo* drug testing in a malignant pleural effusion (MPE) from a non-small cell lung cancer (NSCLC) patient (MPE3). **Fig. 3a** depicts a cellular composition of the MPE at the time of *ex vivo* drug testing, as assessed by flow cytometry. **Fig. 3b** shows a determination of tumour- and immune-specific cytotoxic responses using relative reduction of respective cell types compared to the controls. **Fig. 3c** depicts tumor-specific cytotoxic responses of the MPE to a total of 53 small molecules, antibodies, antibody-drug conjugates, and combinations, all of which are currently approved or in clinical trials for the treatment of NSCLC. **Fig. 3d** depicts the determination of the immunomodulatory effects of antibody-drug conjugates, immunotherapies, and bispecific antibodies in the MPE. This assessment involved analysis of tumor-specific cytotoxicity (1), secreted cytokines (2), and cell-cell interactions (3).

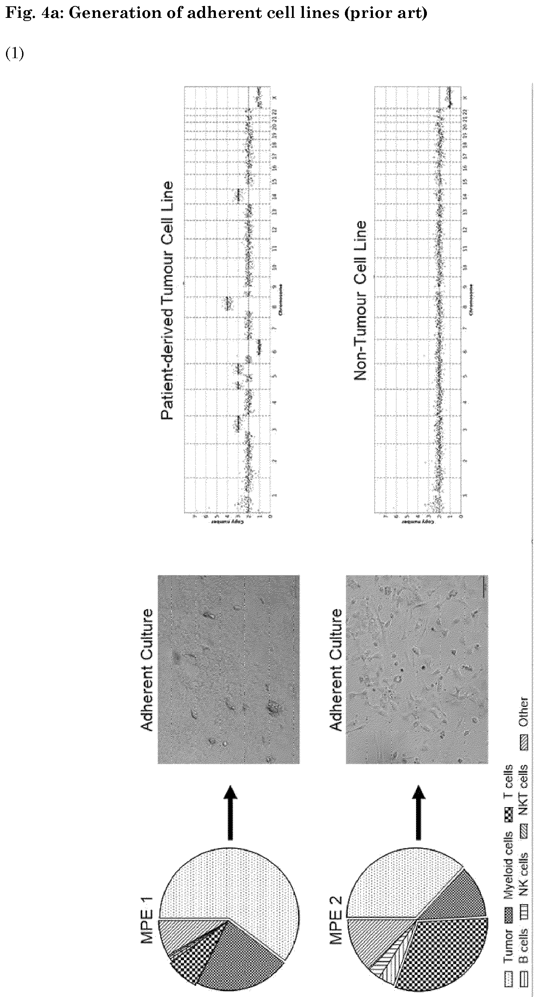

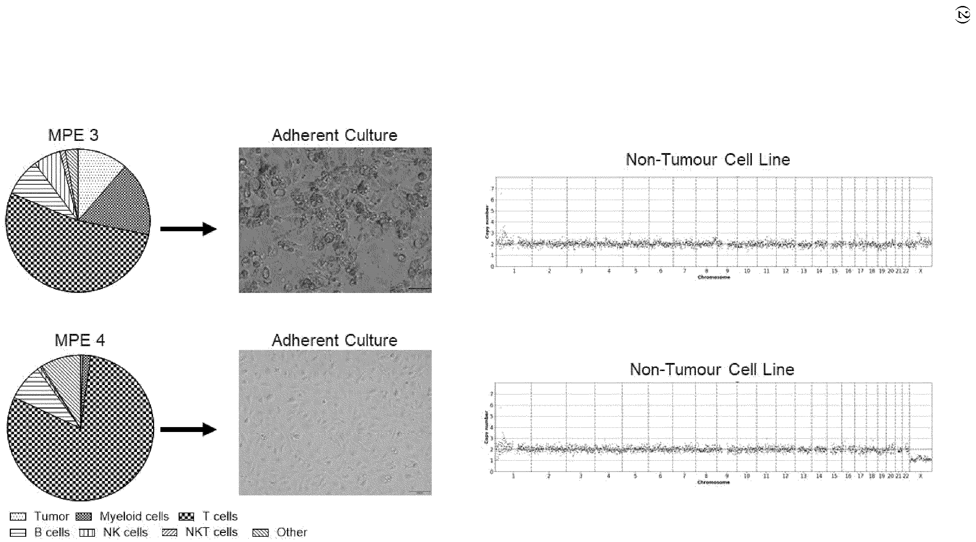

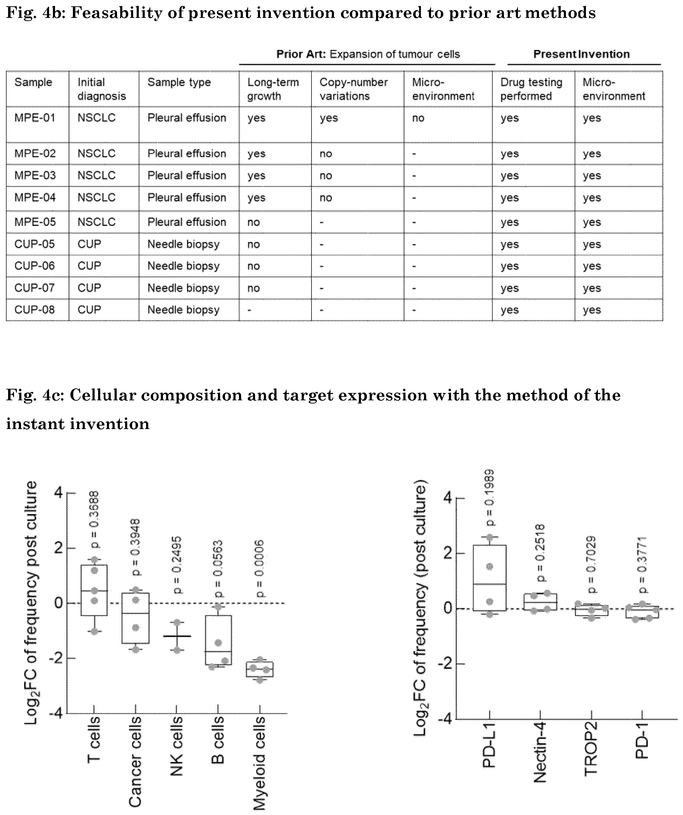

**Fig. 4a** to **4c** depicts that prior art methodologies often lead to insufficient tumor cell outgrowth, the expansion of non-tumor cells, and fail to replicate the components of a cellular microenvironment. **Fig. 4a** shows MPEs from n = 4 NSCLC patients in (1) and (2) that were propagated using state-of-the-art adherent cell culture protocols. The pie charts (left) show the cellular composition of MPEs before tumor cell expansion as assessed by flow cytometry. Brightfield images (middle) display the second passage of adherent cell cultures derived from MPEs. Presence of copy-number variations, assessed by low-pass sequencing, to confirm the tumor origin of these cells (right). **Fig. 4b** depicts a summary table that presents tumor cell outgrowth, malignant origin via copy-number variations, microenvironment presence in the tumor model as generated by prior art methods, and feasibility of drug testing by the present invention. **Fig. 4c** depicts that the present invention preserves the cellular (immune) microenvironment and maintains therapeutic target expression, including immune checkpoint molecules and antibody-drug conjugate targets. Flow cytometric analysis was performed to assess cellular composition and marker expression at day 0 and day 5 of culture in HPLM medium. Log2 fold change (FC) indicates variations in cellular subtypes and marker expression between the time points.

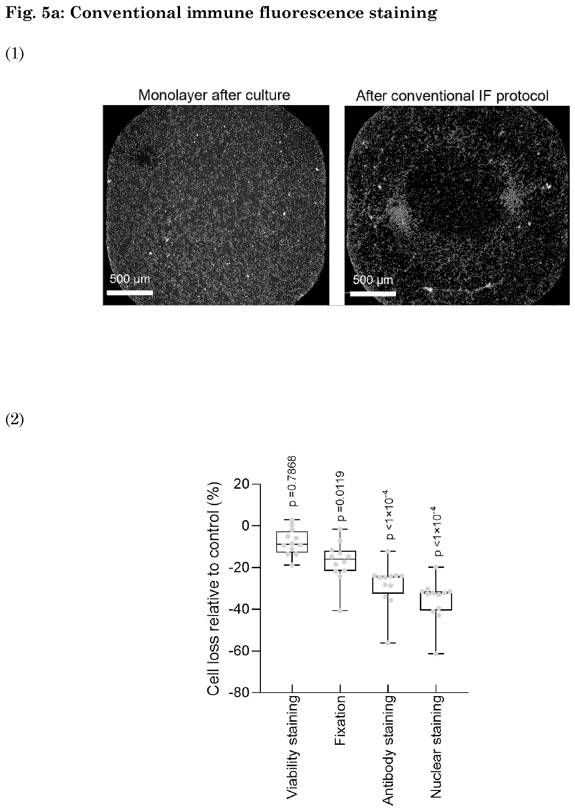

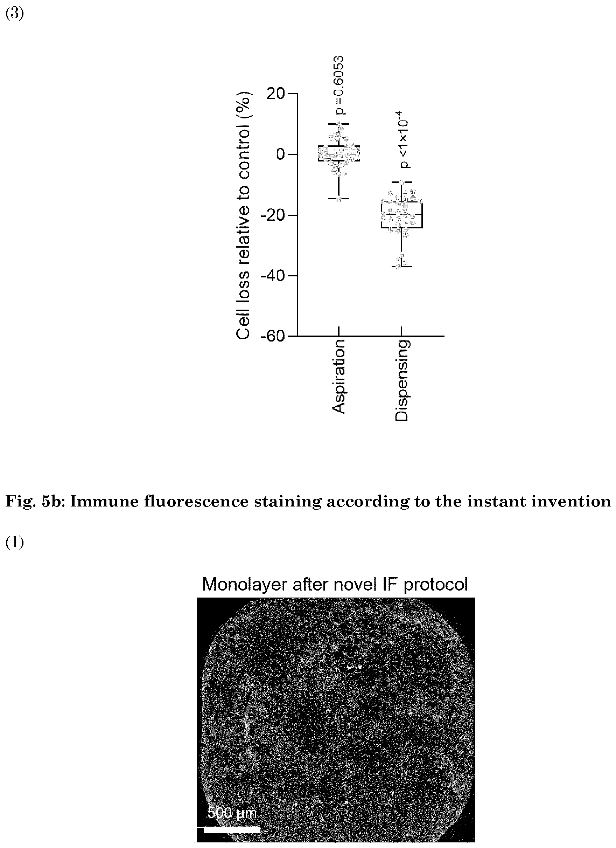

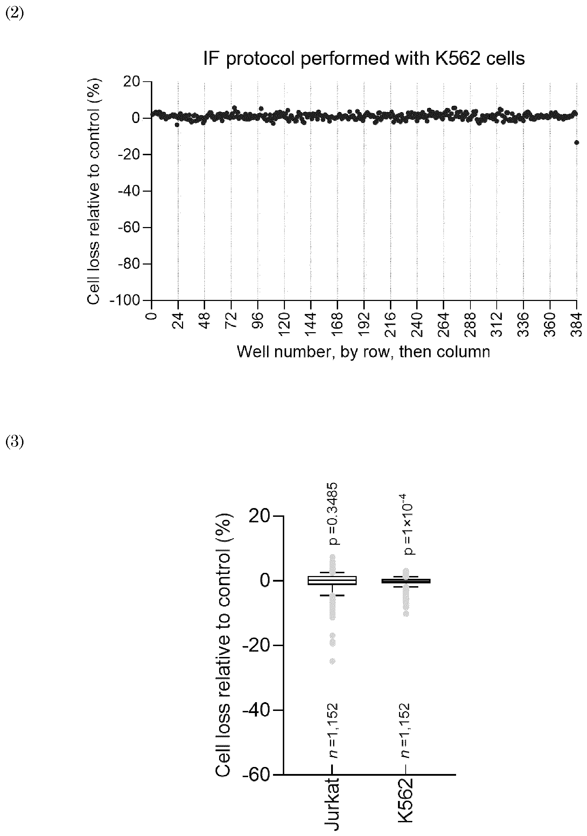

**Fig. 5a** and **5b** show a comparison of prior art immunofluorescence (IF) staining protocols with the present invention. **Fig. 5a** shows a conventional IF staining protocols lead to the loss of non-adherent cells during liquid handling steps. Images (1) display the nuclear (Hoechst 33342) signal of peripheral blood mononuclear cells (PBMCs) in a 384-well plate before and after IF staining. Image analysis was used to evaluate the number of nuclei after each aspiration/dispensing step (2, 3).
**Fig. 5b** shows that the advanced IF staining protocol of the present invention significantly reduces the loss of non-adherent cells during liquid handling. The image (1) shows an intact monolayer of PBMCs following the protocol. This result was confirmed using two non-adherent cell lines, Jurkat and K562, across a total of six 384-well plates. A representative plate with cell loss is shown (2), and cell loss across n=1,152 wells in both cell lines is depicted (3).

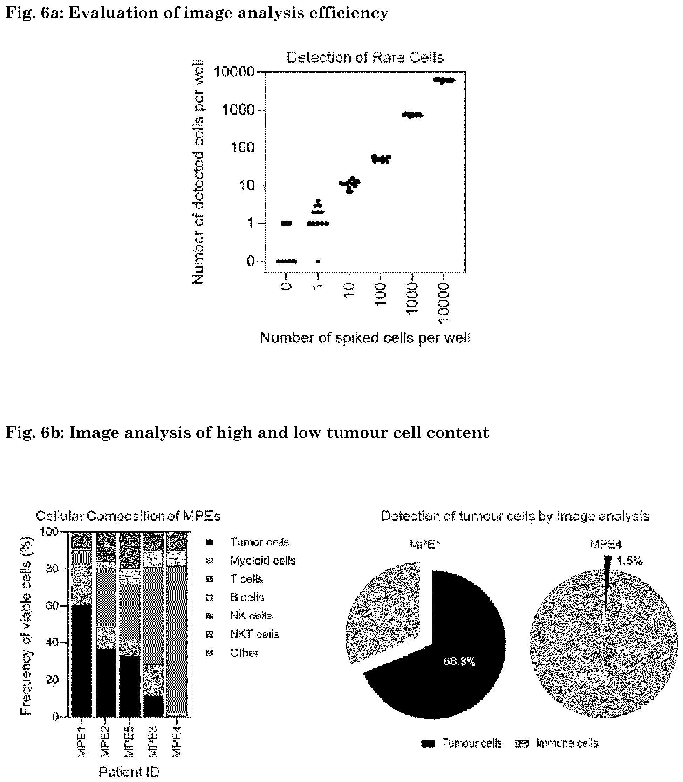

**Fig. 6a** and **6b** depict that image analysis enables highly specific cell detection for the maximum recovery of events for analyzing minimal samples and samples containing rare cells. **Fig. 6a** depicts the evaluation of the image analysis detection limit. Patient-derived lymph node single cell suspensions were spiked with defined numbers of MCF7 tumor cells using FACS sorting (x-axis). The IF protocol of the present invention was applied, and cells were detected through image analysis (y-axis). **Fig. 6b** shows a cellular composition of MPEs from n = 5 NSCLC patients (left), showcasing the significant variability in cancer cell fractions among patients. Image analysis facilitates efficient detection of cancer cells in samples with both high and low tumor cell content (right).

### Detailed description of the invention

The present invention refers to a method for testing an agent, in particular a therapeutic agent, in a tumour sample. The *ex vivo* method of the invention allows in particular determining whether a subject suffering from a tumour such as a primary tumour and/or a metastatis is responsive to the administration of the agent such as a therapeutic agent. The tumour sample is for example obtained from a human or an animal (e.g., mouse, rat, rabbit, goat, dog etc.). It is for example a native animal tumour or a human tumour grown in an animal (e.g., PDX tumour).

The method of the present invention is used for example for the prediction of the responsiveness of a patient on the administration of an agent as well as the testing of the effects of an agent in preclinical studies.

The method of the present invention is highly advantageous for example due to:
- recovery of tumor cells as well as the autologous cellular microenvironment (immune cells) for example by digestion of needle biopsies,
- omitting the pre-expansion of tumor cells and thus, preserving the native cellular composition of the tumour sample,
- high cell recovery during an automated immunofluorescence staining protocol which preserves non-adherent cells for analysis (immune cells) for example by minimizing washing steps, using gentle liquid aspiration techniques that leave a protective fluid layer over the cell monolayer, employing precise pipetting to reduce disturbance and/or maintaining monolayer integrity during staining,
- imaging of all seeded cells by whole-well, z-stack high-content imaging,
- detection and analysis of cells irrespective of their morphology, e.g., size, (non)adherence, immune, cancer for example through an adaptable image analysis that leverages classical and/or deep learning-based segmentation methods, facilitating the recovery and analysis of a maximal number of imaged cells from minimal samples,
- determination of cell-type specific effects by leveraging healthy cells of a sample as an internal control, and/or
- assessment of immunomodulatory effects of an agent as a combination of cancer-specific growth and viability effects, cell-cell contacts between immune cells and tumor cells and expressed cytokines, representing multiple, typically temporally dispersed immunomodulatory outcomes-such as immune activation, inflammation, formation of an immunological synapse between immune and tumor cells, and tumor cell killing-within a single endpoint.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention. The term *"about"* refers to a figure indicated and +/- 10 % of this figure.

In the method of the present invention the effect of an agent, in particular of a therapeutic agent, is tested on a tumour sample. The therapeutic agent is for example selected from the group consisting of a small molecule, a biopharmaceutical such as an antibody, an antibody-drug conjugate, an oligonucleotide, an oncolytic virus, a Chimeric Antigen Receptor (CAR)-T cell, a CAR-NK cell, a CAR-macrophage cell, a Tumour-Infiltrating Lymphocyte (TIL), and a combination thereof. The therapeutic agent is for example an immune therapeutic or a chemotherapeutic.

The effect of the agent tested in the method of the present invention is an effect on tumor cell growth and/or tumor cell viability, in particular an inhibitory effect. This is for example the inhibition of the tumor cell growth, the expression of a marker for cell death and/or apoptosis, in addition or alternatively, the inhibition or upregulation of the expression of a diagnostic molecule and/or therapeutic target, or in addition or alternatively the inhibition of metabolic activity of a primary tumor and/or a metastasis, in particular of the primary tumour cells and/or metastasis cells.

The therapeutic target is for example a biological molecule (such as a protein, an enzyme, a nucleic acid), or a pathway, that when directly or indirectly targeted by an agent such as a therapeutic agent, improves the condition of a disease or a disease process. The diagnostic molecule is for example a biological molecule (such as a protein, an enzyme, a nucleic acid) or a pathway, that indicates whether a therapeutic agent improves or worsens the condition of a disease or disease process.

The sample is neither cultivated nor expanded before the preparation of the tumour sample for the testing of the agent. The preparation of the tumour sample for the testing is a mechanical treatment and/or an enzymatic digestion of the tumour sample. The mechanical treatment comprises for example mincing the tumour sample in pieces, centrifuging the tumour sample, disaggregating the tumour sample, shearing the tumour sample, homogenizing the tumour sample, filtering the tumour sample, triturating the tumour sample, density gradient centrifugation of the tumour sample or a combination thereof. For the enzymatic digestion of the tumour sample for example collagenase, hyaluronidase, DNAse I, trypsin, dispase, lipase, liberase or a combination thereof is added to the tumour sample. After the mechanical treatment and/or an enzymatic digestion the cells of the tumor sample form a single cell suspension.

Viability of the cells of the tumour sample is determined after mechanical treatment and/or enzymatic digestion for example in the single cell suspension, before the cells are settling or when the cells are settled on a surface such as a vessel. Thus, the viability of the cells of the tumour sample including viable immune and/or tumour cells is determined a first time before the incubation of the cells with the agent. Viability of the cells is determined another time during and/or after the incubation of the cells with the agent. Alternatively, viability of all cells of the tumour sample is measured a first and/or a second time. This is measuring a so called bulk viability.

Viability of the cells is determined for example by determining ATP or markers of a healthy cell function such as metabolism, DNA synthesis, cell division, membrane integrity, an apoptosis marker or a combination thereof.

In particular, viability of the cells is determined for example by determining the loss of membrane integrity (e.g., a membrane-impermeant DNA dye such as trypan blue, propidium iodide or 7-Aminoactinomycin (7-AAD)), the presence of externalized phosphatidylserine (e.g., Annexin V conjugated to a fluorophore), caspase-3 and/or caspase-7 activity via a cleavable peptide-fluorophore substrate (e.g., a four amino acid peptide (DEVD)-based dye), cleaved (activated) caspase-3, cleaved (activated) caspase-7, cleaved (activated) caspase-8 and/or cleaved (activated) caspase-9 via a fluorophore-conjugated antibody specific to the cleaved form, cleaved Poly (ADP-ribose) polymerase (PARP) using an antibody specific to the caspase-cleaved epitope, and/or mitochondrial membrane potential changes.

Optionally, the expression of a diagnostic molecule and/or a therapeutic target is measured in the tumor sample, preferably the single cell suspension, by flow cytometry and/or sequencing of nucleic acid such as DNA and/or RNA of tumour cells and/or immune cells prior to the incubation of the cells with the agent. Sequencing of nucleic acid of all cells of the tumour sample is a so called bulk sequencing, measuring nucleic acid of tumour cells and/or immune cells separately is a so called single cell sequencing. The biomarker and/or the therapeutic target are optionally measured in case of a higher amount of the tumour sample. The diagnostic molecule and/or the therapeutic target is one or more of for example PD-L1, PD-1, CTLA-4, TIGIT, TIM-3, LAG-3, ICOS, VISTA, CD112R, BTLA, GITR, NKG2A, HER2 (ERBB2), EGFR, ALK, ROS1, MET, BRAF, KRAS, NRAS, PSMA, GPC3, CEA, CA19-9, CA125, MUC1, WT1, NY-ESO-1, mesothelin, VEGF, FGFR1, FGFR2, FGFR3, RET, NTRK1, NTRK2, NTRK3, PARP, BRCA1, BRCA2, Nectin-4, TROP2, Mesothelin, GPNMB, CD70, CEACAM-5, LIV-1, HER3, c-Met, or a combination thereof.

The number of dying cells and/or dead cells after incubation with the agent is determined for example by dying the cell nucleus with 4', 6-diamin-2-phenylindol (DAPI) or 2'-[4-ethoxyphenyl]-5-[4-methyl-1-piperazinyl]-2,5'-bi-1H-benzimidazole trihydrochloride trihydrate (Hoechst 33342). In addition or alternatively, a marker for dying cells or dead cells is selected from the group consisting of a labelled antibody, a labelled enzyme substrate, a fluorescent dye, a radioactive dye and a combination thereof. Viability of cells is determined via determination of dying and/or dead cells.

The tumour sample is taken from a primary tumour and/or a metastasis. The tumor sample comprises mainly, but not exclusively, tumor cells and immune cells. The tumour cell content of a tumour sample varies for example dependent on the tissue and/or origine of the tumour. The tumour cells are not isolated or propagated from the sample in the method of the present invention for example to concentrate the number of tumor cells. The method of the present invention is very sensitive that a low number of cells from the tumor cell sample is sufficient to get a highly reliable result of the effect of the agent on the tumor cell.

The effect of the agent is for example tested on a fresh tumour sample. The sample is not frozen after the biopsy and thawed before the testing of the agent to avoid loss of cells and cell damages. Alternatively, the tumor sample, in particular a liquid biopsy such as a malignant effusion, e.g., pleural effusion, pericardial effusion or ascites, is frozen, e.g., cryopreserved, for example after taking the biopsy, and thawed for performing the method of the present invention.

The agent is tested on the tumour sample in the method of the present invention about 1 to 48 h, about 1 to 36 h, about 1 to 24 h, 2 to 20 h, 3 to 15 h, 5 to 10 h or 1, 2, 3, 4, 5, 6, 7, 8, 9, 10, 11, 12, 13, 14, 15, 16, 17, 18, 19, 20, 21, 22, 23, 24, 36 or 48 h after taking the biopsy. The biopsy is for example a needle biopsy, a fine needle biopsy or an effusion such as a malignant effusion, e.g., pleural effusion, a pericardial effusion or ascites. Performing the method of the present invention, the effect of the agent on the tumour sample is received within 1 to 10 days, 3 to 9 days, 4 to 8 days, 5 to 6 days, 4 to 5 days or 3 to 4 days, or within 1 day, 2 days, 3 days, 4 days, 5 days, 6 days, 7 days, 8 days, 9 days or 10 days, preferably 3 days or 5 days.

The method of the present invention for testing the effect of an agent on a tumour sample without cultivation or expansion of the tumour sample prior to the following steps comprises
a) mechanical treatment and/or enzymatic digestion of the tumour sample to produce a single cell suspension,
b) determining viability of the cells of the single cell suspension and seeding the single cell suspension on a test vessel for 15 to 60 min at room temperature for cell settling in the test vessel,
c) adding the agent to the settling and/or settled cells and incubating the cells on the test vessel with the agent at a single concentration or varying concentrations at 25-40 °C for 1 to 10 days,
d) collecting a sample of the supernatant from the test vessel for analysing cytokines,
e) staining dying and/or dead cells in the test vessel,
f) fixing cells in the test vessel for performing immune fluorescence to determine a tumour marker, a marker for an immune cell and a marker for the cell nucleus such as 4', 6-diamin-2-phenylindol (DAPI) or 2'-[4-ethoxyphenyl]-5-[4-methyl-1-piperazinyl]-2,5'-bi-1H-benzimidazole trihydrochloride trihydrate (Hoechst 33342),
g) determining the number of viable immune and/or tumour cells in the test vessel during and/or after incubation,
h) calculating a tumour-specific cytotoxic effect of the agent based on the specific reduction of the number of viable tumour cells in relation to the number of viable immune cells and/or
i) determining an immune modulatory effect of the agent based on the interaction of immune cells with tumour cells and/or optionally based on the results of the cytokine analysis of step d).

The single cell suspension of the tumour sample for testing the agent has for example a concentration of 0.01 to 1×10⁶ cells/ml, 0.0125 to 1×10⁸ cells/ml, 0.016 to 1×10⁶ cells/ml, 0.1×10⁶ to 0.5 cells/ml, 0.0125 to 1×10⁶ cells/ml, 0.1 ×10⁶ cells/ml, or 1 ×10⁶ cells/ml.

The method of the present invention is particularly advatageous for testing the effect of an agent, such as a therapeutic agent, on a metastasis. A metastasis is often not surgically removed. As a result, only biopsy material containing a limited number of cells is obtained. The method of the present invention effectively utilizes this small number of cells without requiring purification or isolation of tumor and/or metastasis cells, making it ideal for these scenarios.

Biopsies originating from metastasis of a patient suffering from systemic cancer for *ex-vivo* drug response assays provide a higher predictive value for the treatment efficacy of an anti-cancer therapy than prevailing medical diagnostic procedures, for those cancer patients for whom all therapeutical options have being fully exhausted or for whom a cancer of unknow primary (CUP) has been diagnosed. The method of the present invention allows the determination of a tumour marker and/or a marker of an immune cell. The tumour marker is for example selected from the group consisting of EpCAM, CK1, CK2, CK4, CK5, CK6, CK7, CK8, CK10, CK13, CK14, CK17, CK18, CK19, CK20, E-Cadherin, EMA, TTF-1, Napsin A, p63, p40, p53, GATA3, Mammaglobin, GCDFP-15, CDX2, SATB2, PAX8, Uroplakin II, Uroplakin III, PSA, NKX3.1, Prostein, Claudin-4, Ki-67, CD56, melanoma-associated chondroitin sulfate proteoglycan (MCSP), gp100and a combination thereof. The marker for the immune cell is for example selected from the group consisting of CD45, CD3, CD4, CD8, CD19, CD20, CD45, CD14, CD16, CD56, CD25, CD127, CD11b, CD11c, CD15, CD33, CD34, CD38, CD45RA, CD45RO, HLA-DR, CD66b, CD27, CD28, CD62L, CCR7, CXCR3, CXCR5, FOXP3, CD68 and a combination thereof.

The method of the present invention comprises generally the steps of patient sample processing, sample seeding, application of automated immuno-fluorescence, imaging, image analysis, classification of cells, calculation of cytotoxic drug responses and/or calculation of immunomodulatory responses.

In the patient sample processing, needle biopsies are taken from a tumour and/or a metastasis (e.g. bone, lung) of known or unknown primary tumour. The biopsy is for example transported in 0.9% NaCl.

The biopsy is for example processed by mechanical treatment and/or enzymatic digestion to produce single-cell suspensions. The suspension is seeded to a vessel such as a cell culture or Petri dish, or a well plate for high-throughput imaging. Red blood cell removal is omitted due to the low cell yield from biopsy such as needle biopsy or fine needle biopsy. For samples with viability below 70%, dead cells are removed for example by bead-based separation (e.g., Dead Cell Removal Kit, Miltenyi Biotec). Cells from metastatic needle biopsies are preferably used freshly for downstream applications to minimize cell loss associated with freeze-thaw cycles. In case of a pleural effusion, the mechanical treatment is for example the centrifugation of the effusion and the cell pellet is resuspended to form the single cell suspension.

The dissociation of needle or fine needle biopsy tissue enables maximal recovery of tumor cells as well as microenvironment cells, and allows for seeding of cells in a monolayer in a vessel such as a well plate for high-content imaging. Cells may re-aggregate (i.e., form 3D-structures) during e.g., 5 days of *ex vivo* culture in a patient-dependent manner.

In the sample seeding step after mechanical treatment and/or enzymatic digestion, the needle or fine needle biopsy samples are centrifuged and resuspended in a suspension medium. The suspension medium is for example Human Plasma-like Medium (HPLM, i.e., 10% human AB serum, 1×2-HBA, 100 U/mL Penicillin, 0.1 mg/mL Streptomycin in Human Plasma-Like Medium). The cells in the single cell suspension are seeded at a vessel for example at a concentration of 0.1×10⁶ cells per mL which is for example 50 µL of cell suspension per well of a multi-well plate such as a 384-well plate. The final concentration is for example 5,000 cells per well. If the single cell suspension contains for example >80% of immune cells such as lymphocytes, cells are seeded at a higher number to increase density, e.g., 10,000 or 15,000 cells per well.

The agent to be tested, in particular a therapeutic agent, such as an antibody, antibody-drug conjugates (ADC), an oligonucleotide, an oncolytic virus, a Chimeric Antigen Receptor (CAR)-T cell, a CAR-NK cell, a CAR-macrophage cell, a Tumour-Infiltrating Lymphocyte (TIL), or a combination thereof is added to the cells settling and/or seeded to the vessel such as a well. The agent is added to the cells for example either at single concentrations (maximal plasma concentration of corresponding agent) or at a dose response curves having e.g., 2 to 7 different doses.

In the present method also negative and positive controls are tested for example at least 10% of wells of a multi-well plate comprise negative control wells (e.g. DMSO, PBS, Isotype) and/or positive control wells (e.g., Staurosporine and/or Benzethonium chloride). Agent(s) and controls are dispensed in a randomized layout to avoid vessel effects. After administration of the agent to be tested on the vessel such as a dish or well, the vessel(s) are sealed for example with breathable sealing tape to reduce plate effects and incubated.

In the step of the automated immune fluorescence vessels are removed from the incubator for example after 1 to 10 days, 3 to 6 days, preferably 3 or 5 days of incubation. During or after the incubation, culture supernatant is collected for the analysis of cytokines (e.g., using LEGENDplex multiplex immunoassay, BioLegend). Analysed cytokines are for example IL-4, IL-2, IP-10, IL-1β, TNF-α, MCP-1, IL-17a, IL-6, IL-10, IFN-y, IL-12p70, IL-8, Free active TGF-β1, Fas, FasL, Granzyme A, Granzyme B, Perforin, Granulysin, Arginase, TARC, IL-1RA, IL-12p40, and/or IL-23.

An automated and optimized liquid handling protocol as used in the method of the present invention for example prevents the loss of non-adherent cells during immunofluorescence. The immunofluorescence protocol consists for example of the following steps: First, a dead cell staining reagent is added directly to the cells of the tumour sample following incubation with the agent, without aspirating the incubation medium. Next, a fixation solution is introduced directly into the incubation medium containing cells of the tumour sample after the dead cell staining, again without aspiration of the incubation medium. The fixation solution containing medium is then gently removed, e.g., using a pipetting robot or plate washer. A residual volume of the medium is left, e.g., 10-20 µL, 15-20 µL, or 10-15 µL, creating a protective fluid layer over the cells of the tumour sample to safeguard the monolayer from disturbance during liquid handling.

Subsequently, a blocking solution is gently dispensed at a fixed pipetting height, for example with the pipette tip positioned at the edge of the well to reduce fluid turbulence. The blocking solution is removed afterwards, and the staining solution with a labelled antibody and the nuclear staining reagent is dispensed in the same manner. The antibody is for example fluorescently or radioactvely labelled. After incubation, the antibodies are washed by removing and dispensing Dulbecco's Phosphate Buffered Saine (DPBS), e.g., twice. This protocol is designed to minimize disturbance to the cell monolayer, thereby maintaining cell integrity throughout the procedure. This approach is critical for a biopsy with minimal material because it reduces cell loss, enabling the detection and analysis of a sufficient number of events. Since non-adherent cells are predominantly composed of immune cells (e.g. lymphocytes), the protocol is critical for the testing of immunomodulatory (bio)pharmaceuticals.

Imaging is based for example on fluorescence microcopy such as automated fluorescence microscopy. Cells are imaged using for example a 20x water-objective or 20x air-objective, capturing non-overlapping fields for example 5x5 non-overlapping fields so that the whole well surface is covered. Z-stacks are measured; the number of measured z-stacks is adapted individually for each sample. It is measured for example in 5 µm distances. The images are sequentially taken for example from the Brightfield/Phase contrast, DAPI, Alexa Fluor 594, and Alexa Fluor 647 channels, optionally with non-overlapping lasers and bandpass filter sets to capture the used fluorophores to stain the markers.

High-content imaging of the whole vessel such as a dish or well in z-stacks depicts all seeded cells and generates a maximum number of events for analysis for minimal samples such as biopsies.

For the image analysis precise parameters of the image analysis are adapted for example on a per-sample basis to account for variations in intensity, morphology, and/or cell growth.

In case of z-stacks, the fluorescent channel images are for example merged using maximum projection algorithm (i.e., minimum projection for brightfield images). Optionally, for each individual single-channel image, illumination correction is performed using for example a Gaussian or polynomial function.

For quick visual evaluation and/or segmentation, all raw and/or corrected images of each vessel are optionally for example rescaled, merged, and colorized. Images of markers such as nuclear staining (e.g., DAPI), Dead Stain (e.g, Image-IT Dead Green Viability), immune cell stain (e.g. CD45-PE/Dazzle 594), and/or tumor cell stain (EpCAM-APC) are segmented for example using classical adaptive threshold-based algorithms (e.g. Otsu algorithm) and/or neural network based methods (e.g. Cellpose (Stringer et al. 2021, Pachitariu et al. 2022)) and/or artificial intelligence based methods.

DAPI signal is optionally used as seeds along with a propagate and/or dilate function to detect dividing lines between clumped objects. Cell debris and background artifacts are optionally filtered using a size and/or intensity threshold for the detected objects. Optionally, segmentation quality is visually evaluated by overlaying segmentation masks on the vessel images. Intensity, texture, and shape features (e.g. mean intensity, integrated intensity, standard deviation of intensity, median intensity, maximum or minimum intensity, intensity distribution, area, perimeter, eccentricity, solidity, form factor, extent, axis lengths, radial distribution of intensity, granularity) are measured for example from nuclei, immune, and tumour cells. Object distances and/or neighborhoods are for example measured from tumour-immune and immune-immune objects, respectively. Optionally, single cell crops for immune and/or tumour cells are for example generated based on their respective segmentation masks. View maps of the overall vessel, such as dish or well, of various extracted features such as mean intensity, cell counts per well, mean absolute deviation intensity, etc. are generated to visually check and detect for vessel effects. Outlier wells exhibiting abnormally high or low intensities or cell numbers or intensities are for example removed if the observed pattern is attributed to technical errors, such as liquid handling artifacts.

Marker positivity of cells (masks) and/or viability of cells is for example determined on the basis of manual gating, dimensionality reduction methods (e.g., UMAP or t-SNE), and/or machine learning/Al-based methods, and results are for example curated based on negative and positive controls.

The method of the present invention allows detection of tumor and immune cells of different morphologies (e.g., adherent, clusters, suspension) for example due to the per sample adaptable and/or hybrid approach of combining classical and deep learning-based segmentation methods. This increases efficiency of cell detection and enables measurement of minimal samples.

The *ex vivo* approach fully omits tumor cell enrichment or outgrowth of tumor cells (e.g., generation of cell lines, patient-derived organoids, PDX) and therefore includes the autologous cellular microenvironment of the tumor/metastasis in the native composition. The presence of autologous immune cells enables not only testing of immunomodulatory (bio)pharmaceuticals, like immune checkpoint blockade and antibody-drug conjugates, but further harbors for example an internal healthy cell to determine cancer-specific vs. general toxicity of agents. The cell seeding protocol ensures homogenous distribution of cells for imaging.

The calculation of the tumour cell density in a vessel is performed based on the following formula (1): $Tumour cell density = \frac{Viable tumour cells per vessel}{Viable tumour cells per vessel + Viable immune cells per vessel}$ (1)

The calculation of the cytotoxic agent response is for example based on the following formula (2): $Tumour specificity score = 1 - \frac{Tumour cell density \left(agent\right)}{Tumour cell density \left(control\right)}$ (2) wherein a tumour specificity score > 0 indicates a specific decrease in tumour cell fraction compared to the healthy cell (immune cells) fraction and an untreated/solvent/isotype control. Furthermore, the effect is optionally specified by comparing the relative reduction of tumour and/or immune cells compared to an untreated/solvent/isotype control following formulas (3) and/or (4): $Relative Reduction \left(tumour\right) = \frac{Viable tumour cells per vessel \left(agent\right)}{Viable tumour cells per vessel \left(control\right)} - 1$ (3) and/or $Relative Reduction \left(immune\right) = \frac{Viable immune cells per vessel \left(agent\right)}{Viable immune cells per vessel \left(control\right)} - 1$ (4) wherein a relative reduction <0 indicates an inhibitory effect of an agent to the tumour and/or immune cell and wherein a relative reduction >0 indicates an proliferative effect of an agent to the tumour and/or immune cell.

The present invention results in the measurement of not only bulk viability, but specific effects of agents on tumor cells by comparing these effects to the microenvironment.

For the calculation of an immune modulatory response, cell-cell interactions, e.g., between tumour-immune cells, such as EpCAM-CD45 cells, are detected and evaluated. To evaluate tumour-immune cell contacts for example the average number of immune cells are measured that are directly in contact and/or in proximity/neighborhood with one tumour cell per well. Secondly, the total percentage of for example tumour cells is measured that have any contact with immune cells out of all tumour cells per well.

Optionally, further combinations of dead vs. viable cell contacts are also quantified. Alternatively or in addition, similar combinations also for other interactions such as immune-immune cell interactions, such as CD45-CD45 cells are assessed.

An immunomodulatory effect of an agent is further specified by the cytokines measured in the supernatant. The expression of inflammatory or immunostimulatory cytokines, such as IL-2, IL-1β, TNF-α, MCP-1, IL-17a, IL-6, IFN-y, IL-12p70, and IL-8, in comparison to the respective control vessels, suggests the efficacy of an immunomodulatory agent. Conversely, the expression of antiinflammatory or immunosuppressive cytokines, such as free active TGF-β1, IP-10, and IL-4, for example suggest inefficacy. Additionally, the expression of cytokines in untreated cells for example provide insights into the immune landscape, indicating whether the microenvironment is immune-active ("hot") or immune-inactive ("cold"). Moreover, cytokine expression is used for example to trace effects back to specific cell types, enabling a deeper understanding of the cellular response to the agent (e.g. IL-2 to CD4/CD8 T cells). Furthermore, results are correlated for example to expression of biomarkers/therapeutic targets as assessed by flow cytometry prior to the assay and/or after the assay.

In the method of the present invention, the combination of cancer-specific growth and viability effects, cell-cell contacts between immune cells and tumor cells, and expressed cytokines serve as a surrogate for immunomodulatory effects. This approach enables the assessment of multiple, typically temporally dispersed immunomodulatory outcomes-such as immune activation, inflammation, formation of an immunological synapse between immune and tumor cells, and tumor cell killing-within a single endpoint.

The present invention further refers to a kit for testing the effect of an agent, particularly a therapeutic agent on a tumour sample such as the sample of a primary tumour and/or a metastasis. The effect of the agent on the tumour cell is tested with the kit based on the method of the present invention.

The kit comprises a vessel, a tumour marker, a marker for an immune cell, a marker for cell viability and a marker for the cell nucleus. The vessel is for example a dish such as a Petri dish or a well such as a multi-well plate, e.g., a 384 well-plate.

The marker for the cell nucleus is for example 4', 6-diamin-2-phenylindol (DAPI) or 2'-[4-ethoxyphenyl]-5-[4-methyl-1-piperazinyl]-2,5'-bi-1H-benzimidazole trihydrochloride trihydrate (Hoechst 33342).

The tumour marker is for example selected from the group consisting of EpCAM, CK1, CK2, CK4, CK5, CK6, CK7, CK8, CK10, CK13, CK14, CK17, CK18, CK19, CK20, E-Cadherin, EMA, TTF-1, Napsin A, p63, p40, p53, GATA3, Mammaglobin, GCDFP-15, CDX2, SATB2, PAX8, Uroplakin II, Uroplakin III, PSA, NKX3.1, Prostein, Claudin-4, Ki-67, CD56, melanoma-associated chondroitin sulfate proteoglycan (MCSP), gp100and a combination thereof.

The marker for the immune cell is for example selected from the group consisting of CD45, CD3, CD4, CD8, CD19, CD20, CD45, CD14, CD16, CD56, CD25, CD127, CD11b, CD11c, CD15, CD33, CD34, CD38, CD45RA, CD45RO, HLA-DR, CD66b, CD27, CD28, CD62L, CCR7, CXCR3, CXCR5, FOXP3, CD68 and a combination thereof.

All the compounds, markers, media etc. and their combinations as mentioned for the present method are optionally part of the kit of the present invention.

### Examples

The following examples illustrate different embodiments of the present invention, however, the invention is not limited to these examples.

### Example 1: Testing of therapeutic agents on a needle biopsy obtained from a liver metastasis of cancer of unknown primary origin

The biopsy was transported overnight in sodium chloride solution, weighed, and dissociated into a single-cell suspension. Therefore, the tissue was immersed in tumor digestion medium (0.33 mg/mL Collagenase, 0.1 mg/mL Hyaluronidase, 0.1 mg/mL DNAse I, 1% BSA, 10 mM HEPES, 100 U/mL Penicillin, and 0.1 mg/mL Streptomycin in DMEM/F12), mechanically minced into 1-3 mm³ fragments, and further dissociated through pipetting. The sample was incubated at 37°C for 30 minutes, with periodic resuspension every 10 minutes to aid digestion. Post-digestion, the solution was centrifuged at 300 x g for 10 minutes at room temperature, and the cell pellet was resuspended in 1 mL of trypsin/EDTA solution. After a 5-minute incubation at room temperature, digestion was stopped with 5 mL RPMI1640 containing 10% FCS and the suspension was filtered through a 40 µm cell strainer, with remaining tissue fragments triturated using a 5 mL syringe plunger to enhance cell recovery. After subsequent centrifugation at 300 x g for 5 minutes cells were counted and cellular viability was assessed using Trypan blue staining. Dead cells were removed using dead cell removal beads (Miltenyi Biotec, Bergisch Gladbach) according to the manufacturer's instructions, viable cells were recounted, and resuspended at 0.083 x 10⁶ cells/mL in HPLM medium (10% human AB serum, 50 µM 2-HBA, 100 U/mL Penicillin, and 0.1 mg/mL Streptomycin in Human Plasma-Like Medium). Cells were plated at a density of 617.3 cells/mm² in a Greiner Screenstar 384-well plate (60 µL per well), settled for 45 minutes at room temperature, and therapeutic agents were dispensed in technical quintuplicates using a D300e Digital Dispenser (Tecan Group, Männedorf). Plates were sealed with breathable sealing tape and incubated at 37°C in a 5% CO₂ humidified atmosphere for 5 days. Following drug incubation, 50 nM Image-IT Dead Green viability dye (Thermo Fisher Scientific, Waltham, MA) was added to the wells using a D300e Digital Dispenser, and plates were incubated at 37°C for 30 minutes. For all following steps, liquid handling was performed with an Integra Viaflo equipped with a 384-well head, and aspiration was executed using a BioTek ELx405 Select CW plate washer, following an optimized protocol, retaining 15 µL residual volume after aspiration, with all pipetting steps performed at the well edge. Cells were fixed with 15 µL of 4% paraformaldehyde (PFA) in DPBS directly in wells, followed by a 15-minute incubation at room temperature. The fixative-containing medium was removed, and non-specific binding was blocked with 50 µL Human TruStain FcX^{™} (BioLegend, San Diego, CA; 1:200 in DPBS) for 60 minutes at room temperature. The blocking solution was aspirated, and cells were stained overnight at 4°C with an antibody cocktail: APC anti-human EpCAM (CD326, 1:200), PE-Dazzle^{™} 594 anti-human CD45 (1:200), and DAPI (6 µM) in blocking buffer. Post-staining, cells were washed twice with 50 µL DPBS and stored in 50 µL DPBS at 4°C until imaging. Automated fluorescence microscopy was performed using an Operetta CLS High-Content Analysis System, capturing images with a 20x 0.4 NA air objective. Imaging covered over 90% of the well surface by capturing 4x4 non-overlapping fields, including 5 z-stacks of 5 µM to encompass adherent, non-adherent, and clustering cells. Sequential imaging was conducted with brightfield, DAPI, eGFP, Alexa Fluor 594, and APC channels, using lasers and bandpass filters to ensure non-overlapping channels. Raw .tiff images were transferred and analyzed using CellProfiler 4.2.8 with installed plugins. Fluorescent channel images were merged using maximum projection algorithms (minimal projection for brightfield images), exporting projected images as .tiff files for further analysis. Images of DAPI (nuclei), Alexa Fluor 594 (CD45), and APC (EpCAM) were segmented using CellPose models. Adherent tumor cells were detected using an adaptive threshold-based algorithm (Otsu), with DAPI signal provided as seeds for both methods along with a propagate function to detect dividing lines between clumped objects. Intensity, texture, and shape were measured from nuclei, CD45, and EpCAM cells. Cells with low object intensities or very small/large areas were excluded based on manual gating. Double-positive cells for CD45 and EpCAM were excluded due to potential overlapping signals. Dead cells were identified using manual gating and dimensionality reduction based on dead cell signal positivity. Reduction of tumour and immune cells, as well as tumour-specific cytotoxicity were determined according to formulas (1), (2), (3) and (4).

According to the method of the present invention, 0.676 x 10⁶ single cells were successfully recovered from a needle biopsy, ensuring the availability of a sufficient number of viable cells for comprehensive drug testing **(****Fig. 2a****).** This substantial cell yield allowed for the testing of up to 27 therapeutic agents in technical quintuplicates using a 384-well plate format. The optimized immunofluorescence protocol facilitated automated staining of cells after 5 days of culture, as shown by representative IF images in **Fig. 2b****.** These images enabled precise discrimination between tumor cells (EpCAM-positive) and immune cells (CD45-positive), and further allowed for the differentiation of live and dead cells using a specific marker for cell death, effectively demonstrated for both negative (solvent) control and positive control (benzethonium chloride). Image analyses revealed clear separation of positive and negative controls in both tumor and immune cells, meeting established criteria for assay quality (Markossian et al. 2004; Fig. 2c, left). Furthermore, treatment with representative chemotherapeutics (Fig. 2c, right; gemcitabine, paclitaxel, vinorelbine) led to a specific reduction of tumor cells compared to immune cells, a distinction further quantified by the invention's specificity score. These findings highlight the method's efficacy in drug testing and its potential to advance therapeutic development strategies.

The workflow of the present invention ensures efficient recovery of cells from minimal samples, such as needle biopsies, and enables functional drug testing in these samples using automated immunofluorescence and image analysis in a very short period of time.

### Example 2: Evaluation of the feasibility of the present invention's workflow in malignant pleural effusions (MPEs) from non-small cell lung cancer (NSCLC) patients

MPEs were processed upon receipt by centrifugation at 500 x g for 10 minutes at 4°C. The resulting pellet was resuspended in 2 mL of ACK lysis buffer to induce the lysis of red blood cells, followed by a 2-minute incubation at 4°C. Cells were then washed with 50 mL of DPBS and centrifuged at 500 x g for 10 minutes at 4 °C. The cell pellet was resuspended in DPBS, after which cells were counted and cryopreserved in FBS supplemented with 10% DMSO. Prior to drug testing according to the present invention, cells were thawed, dead cells were removed using dead cell removal beads (Miltenyi Biotec, Bergisch Gladbach) following the manufacturer's instructions, and viable cells were recounted. The cellular composition and viability of the MPE was assessed via flow cytometry. For agent testing, cells were resuspended at 0.083 x 10⁶ cells/mL in Human Plasma-Like Medium (HPLM, as described in Example 1) and seeded at a density of 617.3 cells/mm² in a Greiner Screenstar 384-well plate, pre-spotted with a total of 53 small molecules, antibodies, antibody-drug conjugates, and combinations. Following a 60-minute settling period at room temperature, the plate was sealed with breathable sealing foil and incubated at 37°C in a 5% CO2 humidified atmosphere for 5 days. After incubation, 15 µL of cell culture supernatant were removed from each well, and analyzed using a multiplex immunoassay as per manufacturer's protocols (LEGENDplex HU Essential Immune Response Panel, BioLegend, San Diego). Automated immunofluorescence staining, high-content imaging, and image analysis were performed as detailed in Example 1 to evaluate cellular responses to the therapeutic agents. Object neighbors of tumour cells were measured by overlap of immune cell and tumour cell masks, and the average number of immune cell contacts per tumour cell were analyzed.

A representative malignant pleural effusion (MPE) displayed a diverse cellular composition, including myeloid cells (16.9%), T cells (53.1%), B cells (8.5%), and NK cells (6.1%), alongside 11.3% tumor cells (Fig. 3a). Utilizing the workflow of the present invention, a selection of agents-comprising small molecules, antibodies, antibody-drug conjugates (ADCs), and combinations-currently approved for NSCLC treatment or in clinical trials were evaluated. As indicated by the relative reduction of tumor and immune cells compared to solvent controls, multiple agents demonstrated specific cytotoxic effects targeting cancer cells **(****Fig. 3b****).** Scoring for tumor-specific cytotoxicity identified several agents that effectively reduced the number of viable tumor cells compared to immune cell controls **(****Fig. 3b****),** with the bispecific antibody solitomab and platinum-based chemotherapies emerging as the top hits. Additionally, the efficacy of immunomodulatory agents was analyzed using three measures: tumor-specific cytotoxicity, secreted cytokines, and average immune cell contacts per cancer cell. Strikingly, the bispecific antibody solitomab appeared as a top hit in all readouts, leading to cancer-specific cytotoxicity, increased secretion of inflammatory cytokines, and increased cell-cell interactions, whereas other immunotherapies only increased expression of cytokines.

The present invention successfully facilitated the comprehensive evaluation of therapeutic agents in MPEs from NSCLC patients, demonstrating its robust capability in assessing tumor-specific cytotoxicity, cytokine secretion, and immune cell interactions. This highlights the invention's potential as a powerful tool in advancing targeted cancer therapy evaluations.

### Example 3: Comparison of the efficiency of traditional methodologies, which rely on the expansion of tumor cells prior to drug testing, with the present invention

The cellular composition of MPEs from n = 4 Non-Small Cell Lung Cancer (NSCLC) patients was evaluated using flow cytometry. Tumor cells were cultured under adherent conditions as published in Kodack et al. 2017, with the aim of expanding them for subsequent drug testing, and their origin was confirmed through low-pass sequencing after passages 1-2. Additionally, needle biopsies from n = 4 Cancer of CUP patients were cultured using state-of-the-art patient-derived organoid (PDO) culture conditions, as described by Sachs et al. (2018), to assess the feasibility of expanding these samples for drug testing. In parallel, both MPEs and biopsies were subjected to the drug testing workflow of the present invention. To evaluate the invention's ability to preserve the cellular composition of samples over a 5-day culture period, MPEs were cultured under the conditions outlined in this invention (see Example 1). Cellular composition was reassessed by flow cytometry after the 5-day culture period.

MPEs exhibited a heterogeneous composition of immune cell types, including myeloid cells, T cells, B cells, and NK cells, alongside variable fractions of tumor cells **(****Fig. 4a****,** left). Culturing these cell suspensions under adherent conditions resulted in the growth of adherent cells in all four samples **(****Fig. 4a****,** middle). However, CNV analysis via low-pass sequencing revealed that only one of the four cultures contained tumor cells in passages 1-2, suggesting overgrowth by healthy cells and rendering these cultures unsuitable for drug testing **(****Fig. 4a****,** right). Similarly, attempts to expand tumor cells from CUP biopsies in PDO cultures did not result in tumor cell outgrowth, rendering these samples unusable for drug testing using prior art methodologies. In contrast, the present invention's workflow enabled successful drug testing, meeting quality criteria across all samples, demonstrating its efficacy in overcoming limitations of traditional approaches **(****Fig. 4b****).** Additionally, flow cytometric analysis of MPE cells before and after *ex vivo* culture using the present invention showed conservation of major immune subsets over time, with only myeloid cells being lost due to strong adherence to culture plates **(****Fig. 4c****).** Importantly, the expression of therapeutic targets, such as immune checkpoint molecules (PD-L1, PD-1) and common ADC targets (TROP2, Nectin-4), was maintained after *ex vivo* culture, underscoring the invention's capability to preserve critical therapeutic markers.

Prior art methods focusing on tumor cell propagation frequently result in inadequate tumor cell outgrowth, excessive expansion of non-tumor cells, and fail to maintain the cellular microenvironment's components. In contrast, the present invention demonstrated the feasibility of drug testing across all samples while preserving the cellular composition and maintaining the expression of key biomarkers during the drug testing process.

### Example 4: Comparison of the efficiency of the current invention's immunofluorescence (IF) protocol with prior art protocols, specifically focusing on reducing the loss of non-adherent cells during liquid handling steps

Blood peripheral mononuclear cells (PBMCs), Jurkat cells, and K562 cells, respectively, were stained using Hoechst 33342 according to the manufacturer's protocol (Thermo Fisher Scientific, Waltham, MA) and resuspended in HPLM medium at a concentration of 0.163 x 10⁶ cells/mL. PBMCs were plated at a density of 617.3 cells/mm² in a Greiner Screenstar 384-well plate and allowed to settle for 45 minutes at room temperature. The monolayer of cells was imaged prior to initiating the IF protocol, as well as after key steps and upon completion of the protocol.

The conventional IF protocol involved the following steps:
1. Addition of a viability dye directly to the medium.
2. One washing step with DPBS.
3. Fixation with 25 µL of 4% paraformaldehyde (PFA).
4. One washing step with DPBS.
5. Dispensing of 20 µL of antibody staining solution.
6. One additional washing step with DPBS.
7. Dispensing of 50 µL of DAPI solution.
8. Two final washing steps with DPBS.

Fluid dispensing was conducted using an Integra Viaflo equipped with a 384-well head, while aspiration was performed using a BioTek ELx405 Select CW plate washer. Both dispensing and aspiration were executed in the middle of the well, adhering to prior art methods. The residual volume post-aspiration was minimal, consistent with conventional approaches. In contrast, the IF protocol of the present invention was performed which is based on minimizing washing steps (see Example 1), retaining 10-15 µL residual volume after aspiration, and aspiration/dispensing operations at the well edge.

Performing prior art IF protocols in 384-well plates led to substantial disruption of the monolayer of non-adherent cells, such as PBMCs, creating an empty area in the center of the well, and consequently impeding efficient image analysis **(****Fig. 5a****,** left). This disruption caused significant cell loss during critical steps of the IF protocol, with cell loss reaching up to 40% **(****Fig. 5a****,** middle). Notably, in particular dispensing steps contributed to the loss of cells in these conventional protocols, highlighting the limitations in maintaining cell integrity during liquid handling processes in classical IF protocols. In contrast, the IF protocol developed in this invention maintained an intact monolayer of cells throughout the procedure **(****Fig. 5b****,** left). To demonstrate its suitability for high-throughput screening, the protocol was tested across 1,152 wells (three 384-well plates) using non-adherent leukemia cell lines Jurkat and K562, resulting in no cell loss in either cell line **(****Fig. 5b****,** right).

Unlike prior art IF protocols, the IF protocol of the present invention maintains a stable monolayer of cells throughout the process, minimizing disruption during liquid handling and enabling precise cellular analysis in high-throughput settings.

### Example 5: Assessment of the specificity of the assay in detecting rare cells or minimal samples

A single cell suspension of lymph node cells was prepared using protocols from Ulmer et al. (2018) and resuspended in Human Plasma-Like Medium (HPLM) at 0.163 x 10⁶ cells/mL. Cells were plated at 617.3 cells/mm² in a Greiner Screenstar 384-well plate and allowed to settle for 45 minutes at room temperature. MCF-7 tumour cells were sorted into wells containing lymph node cells using a FACS sorter, from 0 to 10,000 cells per well in log10 steps. After 4 hours of settling, IF staining and image analysis were conducted as detailed in Example 1 to detect tumor cells. To highlight clinical need for cell detection, the cellular composition of MPEs from five NSCLC patients was characterized by flow cytometry. The MPEs with the highest and lowest tumor cell content were processed as described in Example 2, and the fraction of tumour and immune cells was assessed by image analysis

The present invention enabled efficient detection of MCF-7 cells within lymph node cells, successfully identifying as few as 1-10 cells per well (Fig. 6a). This efficiency is vital given the sparse tumor cell content often found in MPEs, highlighting the importance of robust image analysis strategies **(****Fig. 6b****,** left). Furthermore, the invention facilitated the detection of both tumor and immune cells in samples with varying tumor cell content, from very high (MPE1) to very low (MPE4) levels **(****Fig. 6b****,** right).

The method of the present invention demonstrated high specificity in detecting rare cells, showcasing its ability to efficiently recover and analyze a maximal number of imaged cells from minimal samples.

### Embodiments of the invention

1) Method for testing the effect of an agent on a tumour sample without cultivation or expansion of the tumour sample prior to the following steps of:
   a) mechanical treatment and/or enzymatic digestion of the tumour sample to produce a single cell suspension,
   b) determining viability of the cells of the single cell suspension and seeding the single cell suspension on a test vessel for 15 to 60 min at room temperature for cell settling in the test vessel,
   c) adding the agent to the settling and/or settled cells and incubating the cells on the test vessel with the agent at a single concentration or varying concentrations at 25-40 °C for 3 to 10 days,
   d) collecting a sample of the supernatant from the test vessel for determining cytokine expression using a cytokine marker,
   e) staining dying and/or dead cells in the test vessel using a marker for dying and/or dead cells,
   f) fixing cells in the test vessel for performing immune fluorescence to determine a tumour marker, a marker for an immune cell and a marker for the cell nucleus,
   g) determining the number of viable immune and/or tumour cells in the test vessel during and/or after incubation, and/or optionally determining a marker for a therapeutic target and/or diagnostic molecule of viable immune and/or tumour cells or all cells of the tumour sample in the test vessel during and/or after incubation,
   h) calculating a tumour-specific cytotoxic effect of the agent based on the specific reduction of the number of viable tumour cells in relation to the number of viable immune cells and/or
   i) determining an immune modulatory effect of the agent based on the interaction of immune cells with tumour cells and/or optinally based on the results of the cytokine analysis of step d).
2. Method of embodiment 1, wherein the tumour sample has a size of about 1 to 2 mm x 10 to 20 mm.
3. Method of embodiment 1 or 2, wherein the tumour sample is a fresh tumour sample.
4. Method of any one of embodiments 1 to 3, wherein the sample is a biopsy.
5. Method of embodiment 4, wherein the biopsy is a needle biopsy.
6. Method of embodiment 4, wherein the biopsy is a fine needle biopsy.
7. Method of embodiment 4, wherein the biopsy is a liquid biopsy.
8. Method of embodiment 4, wherein the liquid biopsy is a malignant effusion.
9. Method of embodiment 4 or 5, wherein the liquid biopsy is pleural effusion.
10. Method of embodiment 4 or 5, wherein the liquid biopsy is pericardial effusion.
11. Method of embodiment 4 or 5, wherein the liquid biopsy is ascites.
12. Method of any one of embodiments 1 to 11, wherein the liquid biopsy is frozen.
13. Method of any one of embodiments 1 to 12, wherein the single cell suspension has a concentration of 0.01 to 1×10⁶ cells/ml.
14. Method of any one of embodiments 1 to13, wherein the single cell suspension has a concentration of 0.1 ×10⁶ cells/ml.
15. Method of any one of embodiments 1 to 14, wherein the single cell suspension has a concentration of 1 ×10⁶ cells/ml.
16. Method of any one of embodiments 1 to 15, wherein the single cell suspension has a concentration of 0.0125 to 1×10⁶ cells/ml.
17. Method of any one of embodiments 1 to 16, wherein the single cell suspension has a concentration of 0.016 to 1×10⁶ cells/ml.
18. Method of any one of embodiments 1 to 17, wherein the vessel is a dish such as a Petri dish.
19. Method of any one of embodiments 1 to 17, wherein the vessel is a well.
20. Method of any one of embodiments 1 to 17, wherein the vessel is a multi-well plate such as a 384 well plate.
21. Method of any one of embodiments 1 to 20, wherein the single cell suspension is seeded in the test vessel at 1000 to 20000 cells/vessel in step b).
22. Method of any one of embodiments 1 to 21, wherein the single cell suspension is seeded in the test vessel at 1000 to 15000 cells/vessel in step b).
23. Method of any one of embodiments 1 to 22, wherein the single cell suspension is seeded in the test vessel at 1000 to 10000 cells/vessel in step b).
24. Method of any one of embodiments 1 to 23, wherein the single cell suspension is seeded in the test vessel at 1000 to 5000 cells/vessel in step b).
25. Method of any one of embodiments 1 to 20, wherein the single cell suspension is seeded in the test vessel at 5000 to 12000 cells/vessel in step b).
26. Method of any one of embodiments 1 to 23, wherein the single cell suspension is seeded in the test vessel at 10000 cells/vessel in step b).
27. Method of any one of embodiments 1 to 25, wherein the single cell suspension is seeded in the test vessel at 5000 cells/vessel in step b).
28. Method of any one of embodiments 1 to 20, wherein the single cell suspension is seeded in the test vessel at a cell density of 123.5 cells per mm² to 2469.1 cells per mm².
29. Method of any one of embodiments 1 to 20, wherein the single cell suspension is seeded in the test vessel at a cell density of 123.5 cells per mm² to 1234.6 cells per mm².
30. Method of any one of embodiments 1 to 20, wherein the single cell suspension is seeded in the test vessel at a cell density of 123.5 cells per mm² to 617.3 cells per mm².
31. Method of any one of embodiments 1 to 20, wherein the single cell suspension is seeded in the test vessel at a cell density of 617.3 cells per mm² to 2469.1 cells per mm².
32. Method of any one of embodiments 1 to 20, wherein the single cell suspension is seeded in the test vessel at a cell density of 617.3 cells per mm² to 1234.6 cells per mm².
33. Method of any one of embodiments 1 to 20, wherein the single cell suspension is seeded in the test vessel at a cell density of 2469.1 cells per mm².
34. Method of any one of embodiments 1 to 20, wherein the single cell suspension is seeded in the test vessel at a cell density of 1234.6 cells per mm².
35. Method of any one of embodiments 1 to 20, wherein the single cell suspension is seeded in the test vessel at a cell density of 617.3 cells per mm².
36. Method of any one of embodiments 1 to 20, wherein the single cell suspension is seeded in the test vessel at a cell density of 123.5 cells per mm².
37. Method of any one of embodiments 1 to 36, wherein the single cell suspension is seeded on the test vessel for about 45 to 60 min.
38. Method of any one of embodiments 1 to 36, wherein the single cell suspension is seeded on the test vessel for about 30 to 45 min.
39. Method of any one of embodiments 1 to 36, wherein the single cell suspension is seeded on the test vessel for about 15 to 30 min.
40. Method of any one of embodiments 1 to 39, wherein the single cell suspension is seeded on the test vessel at 15 to 45°C.
41. Method of any one of embodiments 1 to 40, wherein the single cell suspension is seeded on the test vessel at 20 to 25°C.
42. Method of any one of embodiments 1 to 39, wherein the single cell suspension is seeded on the test vessel at room termperature.
43. Method of any one of embodiments 1 to 42, wherein the cell settling in the test vessel in step b) is uniform cell settling.
44. Method of any one of embodiments 1 to 43, wherein the test vessel is incubated at 37 °C in step c).
45. Method of any one of embodiments 1 to 44, wherein the test vessel is incubated for 5 to 8 days in step c).
46. Method of any one of embodiments 1 to 44, wherein the test vessel is incubated for 5 days in step c).
47. Method of any one of embodiments 1 to 44, wherein the test vessel is incubated for 3 days in step c).
48. Method of any one of embodiments 1 to 47, wherein the marker for the cell nucleus is 4', 6-diamin-2-phenylindol (DAPI) or 2'-[4-ethoxyphenyl]-5-[4-methyl-1-piperazinyl]-2,5'-bi-1H-benzimidazole trihydrochloride trihydrate (Hoechst 33342).
49. Method of any one of embodiments 1 to 48, wherein the marker for dying and/or dead cells is selected from the group consisting of an antibody, an enzyme substrate, a fluorescent dye, a radioactive dye and a combination thereof.
50. Method of any one of embodiments 1 to 48, wherein the fluorescent dye is a fixable fluorescent dye.
51. Method of any one of embodiments 1 to 48, wherein the antibody is a labelled antibody such as a fluorescent or radioactively labelled antibody.
52. Method of any one of embodiments 1 to 50, wherein the dye is a membrane-impermeant dye to healthy cells such as a DNA specific dye or an amine-reactive dye.
53. Method of embodiment 52, wherein the DNA specific dye is propidium iodide or 7-amino actinomycin D (7-AAD).
54. Method of any one of embodiments 1 to 49, wherein the enzmye substrate is a labelled enzyme substrate such as fluorescent or radioactively labelled.
55. Method of embodiment 54, wherein the enzmye substrate is a cleavable peptide-fluorophore substrate.
56. Method of embodiment 55, wherein the peptide-fluorophore substrate is cleavable by caspase-3 and/or caspase-7.
57. Method of any one of embodiments 1 to 51, wherein the labelled antibody is a fluorophore-conjugated antibody specifically binding to cleaved caspase-3, caspase-7, caspase-8, and/or caspase-9.
58. Method of any one of embodiments 1 to 51, wherein the labelled antibody is specifically binding the caspase-cleaved epitope of cleaved poly (ADP-ribose) polymerase (PARP).
59. Method of any one of embodiments 1 to 50, wherein the fluorescent dye is a potential-sensitive fluorescent dye for detecting mitochondrial membrane potential changes.
60. Method of embodiment 59, wherein the potential-sensitive fluorescent dye is selected from the group consisting of JC-1, tetramethylrhodamine ethyl ester (TMRE), tetramethylrhodamine methyl ester (TMRM), and a combination thereof.
61. Method of any one of embodiments 1 to 60, wherein the marker for dying and/or dead cells is an apoptotic marker.
62. Method of embodiment 61, wherein the apoptotic marker is Annexin V for example conjugated to a fluorophore.
63. Method of any one of embodiments 1 to 62, wherein the tumour marker is selected from the group consisting of EpCAM, CK1, CK2, CK4, CK5, CK6, CK7, CK8, CK10, CK13, CK14, CK17, CK18, CK19, CK20, E-Cadherin, EMA, TTF-1, Napsin A, p63, p40, p53, GATA3, Mammaglobin, GCDFP-15, CDX2, SATB2, PAX8, Uroplakin II, Uroplakin III, PSA, NKX3.1, Prostein, Claudin-4, Ki-67, CD56, MCSP, gp100 and a combination thereof.
64. Method of any one of embodiments 1 to 63, wherein the tumour marker is selected from the group consisting of EpCAM, CK1, CK2, CK4, CK5, CK6, CK7, CK8, CK10, CK13, CK14, CK17, CK18, CK19, CK20 and a combination thereof.
65. Method of any one of embodiments 1 to 64, wherein the tumour marker is EpCAM.
66. Method of any one of embodiments 1 to 65, wherein the tumour marker is CK1.
67. Method of any one of embodiments 1 to 66, wherein the tumour marker is CK2.
68. Method of any one of embodiments 1 to 67, wherein the tumour marker is CK4.
69. Method of any one of embodiments 1 to 68, wherein the tumour marker is CK5.
70. Method of any one of embodiments 1 to 69, wherein the tumour marker is CK6.
71. Method of any one of embodiments 1 to 70, wherein the tumour marker is CK7.
72. Method of any one of embodiments 1 to 71, wherein the tumour marker is CK8.
73. Method of any one of embodiments 1 to 72, wherein the tumour marker is CK10.
74. Method of any one of embodiments 1 to 73, wherein the tumour marker is CK13.
75. Method of any one of embodiments 1 to 74, wherein the tumour marker is CK14.
76. Method of any one of embodiments 1 to 75, wherein the tumour marker is CK17.
77. Method of any one of embodiments 1 to 76, wherein the tumour marker is CK18.
78. Method of any one of embodiments 1 to 77, wherein the tumour marker is CK19.
79. Method of any one of embodiments 1 to 78, wherein the tumour marker is CK20.
80. Method of any one of embodiments 1 to 79, wherein the tumour marker is selected from the group consisting of E-Cadherin, EMA, TTF-1, Napsin A, p63, p40, p53, GATA3, Mammaglobin, GCDFP-15, CDX2, SATB2, PAX8, Uroplakin II, Uroplakin III, PSA, NKX3.1, Prostein, Claudin-4, Ki-67, CD56, MCSP, gp100 and a combination thereof.
81. Method of any one of embodiments 1 to 80, wherein the marker for the immune cell is selected from the group consisting of CD45, CD3, CD4, CD8, CD19, CD20, CD45, CD14, CD16, CD56, CD25, CD127, CD11b, CD11c, CD15, CD33, CD34, CD38, CD68, CD45RA, CD45RO, HLA-DR, CD66b, CD27, CD28, CD62L, CCR7, CXCR3, CXCR5, FOXP3, CD68 and a combination thereof.
82. Method of any one of embodiments 1 to 81, wherein the marker for the immune cell is selected from the group consisting of CD45, CD3, CD4, CD8 and a combination thereof.
83. Method of any one of embodiments 1 to 82, wherein the immune cell marker is CD45.
84. Method of any one of embodiments 1 to 83, wherein the immune cell marker is CD3.
85. Method of any one of embodiments 1 to 84, wherein the immune cell marker is CD4.
86. Method of any one of embodiments 1 to 85, wherein the immune cell marker is CD8.
87. Method of any one of embodiments 1 to 86, wherein the marker for the immune cell is selected from the group consisting of CD19, CD20, CD45, CD14, CD16, CD56, CD25, CD127, CD11b, CD11c, CD15, CD33, CD34, CD38, CD68, CD45RA, CD45RO, HLA-DR, CD66b, CD27, CD28, CD62L, CCR7, CXCR3, CXCR5, FOXP3, CD68 and a combination thereof.
88. Method of any one of embodiments 1 to 87, wherein the tumour marker is EpCAM and the immune cell marker is CD45.
89. Method of any one of embodiments 1 to 87, wherein the immune cell markers are CD45.
90. Method of any one of embodiments 1 to 89, wherein the marker for a therapeutic target and/or diagnostic molecule is selected from the group consisting of PD-L1, PD-1, CTLA-4, TIGIT, TIM-3, LAG-3, ICOS, VISTA, CD112R, BTLA, GITR, NKG2A, HER2 (ERBB2), EGFR, ALK, ROS1, MET, BRAF, KRAS, NRAS, PSMA, GPC3, CEA, CA19-9, CA125, MUC1, WT1, NY-ESO-1, mesothelin, VEGF, FGFR1, FGFR2, FGFR3, RET, NTRK1, NTRK2, NTRK3, PARP, BRCA1, BRCA2Nectin-4, TROP2, Mesothelin, GPNMB, CD70, CEACAM-5, LIV-1, HER3, c-Met and a combination thereof.
91. Method of any one of embodiments 1 to 90, wherein the cytokine marker is selected from the group consisting of IL-4, IL-2, IP-10, IL-1β, TNF-α, MCP-1, IL-17a, IL-6, IL-10, IFN-y, IL-12p70, IL-8, Free active TGF-β1, Fas, FasL, Granzyme A, Granzyme B, Perforin, Granulysin, Arginase, TARC, IL-1RA, IL-12p40, IL-23 and a combination thereof.
92. Method of any one of embodiments 1 to 91, wherein the mechanical treatment is selected from the group consisting of mincing the tumour sample, centrifuging the tumour sample, disaggregating the tumour sample, shearing the tumour sample, homogenizing the tumour sample, filtering the tumour sample, triturating the tumour sample, density gradient centrifugation of the tumour sample or a combination thereof.
93. Method of any one of embodiments 1 to 92, wherein the tumour sample is mechanically minced into pieces of 0.5 to 5 mm³ in a suspension medium, incubated for 10 to 60 min at 20 °C to 40 °C and resuspended every 10 min to form the single cell suspension in step a).
94. Method of any one of embodiments 1 to 93, wherein the tumour sample is mechanically minced into pieces of 1 to 3 mm³ in the suspension medium in step a).
95. Method of any one of embodiments 1 to 94, wherein the minced tumour sample is incubated for 20 to 40 min.
96. Method of any one of embodiments 1 to 95, wherein the minced tumour sample is incubated at 37°C.
97. Method of any one of embodiments 1 to 96, wherein the tumour sample is liquid biopsy such as pleura effusion, pericardial effusion or ascites which is centrifuged in step b), optionally red blood cells are lysed, and the centrifuged cells are resuspended as single cell suspension.
98. Method of embodiment 97, wherein the cell concentration in the single cell suspension is 0.01 to 1×10⁶ cells/ml.
99. Method of embodiment 97 or 98, wherein the cell concentration in the single cell suspension is 0.1 to 1×10⁶ cells/ml.
100. Method of any one of embodiments 97 to 99, wherein the cell concentration in the single cell suspension is 0.0125 or 0.016 to 1×10⁶ cells/ml.
101. Method of any one of embodiments 1 to 100, wherein the suspension medium comprises or consists of human serum and antibiotics in a culture medium.
102. Method of embodiment 101, wherein the suspension medium comprises or consists of 10% human serum (type AB), 100 U/ml penicillin, 0.1 mg/ml streptomycin, 50 µM 2-hydroxybutyric acid (2-HBA) in human plasma-like medium (HPLM).
103. Method of any one of embodiments 1 to 102, wherein the tumour sample or the single cell suspension of the mechanical treatment is enzymatically digested.
104. Method of any one of embodiments 1 to 103, wherein the tumour sample or the single cell suspension of the mechanical treatment is digested with collagenase, hyaluronidase, DNAse I, trypsin, dispase, lipase, liberase and a combination thereof.
105. Method of any one of embodiments 1 to 104, wherein the enzymatically digested tumour sample or single cell suspension is filtered through a strainer and residual tissue fragments on the strainer are triturated to maximize cell recovery. 106. Method of any one of embodiments 1 to 105, wherein the antibody is a labelled with a fluorophore of the group of Spark UV^{™} 387, Brilliant Violet 421^{™}, Spark Violet^{™} 423, Super Bright 436, Pacific Blue^{™}, Brilliant Violet 510^{™}, Spark Violet^{™} 538, Brilliant Violet 570^{™}, Brilliant Violet 605^{™}, Brilliant Violet 650^{™}, Brilliant Violet 711^{™}, Brilliant Violet 750^{™}, Brilliant Violet 785^{™}, KIRAVIA Blue 520^{™}, FITC, Spark Blue^{™} 550, Spark Blue^{™} 574, PerCP, PerCP/Cyanine5.5, PE, PE/Dazzle^{™} 594, PE/Fire^{™} 640, PE/Cyanine5, PE/Fire^{™} 700, PE/Cyanine7, PE/Fire^{™} 810, Spark YG^{™} 581, Spark YG^{™} 593, APC, Alexa Fluor^{®} 647, Spark NIR^{™} 685, Spark Red^{™} 718, Alexa Fluor^{®} 700, APC/Cyanine7, APC/Fire^{™} 750, APC/Fire 810^{™}, Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 635, Alexa Fluor 647, Alexa Fluor 660, Alexa Fluor 680, Alexa Fluor 700, Alexa Fluor 750, Alexa Fluor 790 and a combination thereof.
107. Method of any one of embodiments 1 to 106, wherein dead cells are removed if the cell viability is below 70 % in step b).
108. Method of any one of embodiments 1 to 107, wherein
   a fluorescence signal of the marker is sequentially measured using fluorescence microscopy in channels wherein laser and bandpass filter sets are set to non-overlapping channels.
109. Method of embodiment 108, wherein the fluorescence microscopy is automated fluorescence microscopy.
110. Method of any one of embodiments 1 to 109, wherein a tumour-specific cytotoxic effect of the agent in a vessel is based on a greater reduction in viable tumour cells compared to the reduction in viable immune cells, when compared to a solvent, untreated, or isotype control vessel according to formula 1): $Tumour cell density = \frac{Viable tumour cells per vessel}{Viable tumour cells per vessel + Viable immune cells per vessel}$ (1) wherein a tumour specificity score > 0 indicates a specific decrease in tumour cell fraction compared to an untreated control, and optionally the tumour-specific cytotoxic effect of the agent is further specified according to formulas (2), (3) and/or (4): $Tumour specificity score = 1 - \frac{Tumour cell density \left(agent\right)}{Tumour cell density \left(control\right)}$ (2) $Relative Reduction \left(tumour\right) = \frac{Viable tumour cells per vessel \left(agent\right)}{Viable tumour cells per vessel \left(control\right)} - 1$ (3) and/or $Relative Reduction \left(immune\right) = \frac{Viable immune cells per vessel \left(agent\right)}{Viable immune cells per vessel \left(control\right)} - 1$ (4) wherein a relative reduction <0 indicates an inhibitory effect of an agent to the tumour and/or immune cell.
111. Method of any one of embodiments 1 to 110, wherein cell interactions are calculated as the mean number of direct immune cell contacts per tumour cell in a vessel, when compared to a solvent, untreated, or isotype control vessel.
112. Method of any one of embodiments 1 to 111, wherein the agent is a therapeutic agent.
113. Method of any one of embodiments 1 to 112, wherein the therapeutic agent is a small molecule, a biopharmaceutical, or a combination thereof.
114. Method of any one of embodiments 1 to 113, wherein the biopharmaceutical is selected from the group consisting of an antibody, an antibody-drug conjugate (ADC), an oligonucleotide, an oncolytic virus, a Chimeric Antigen Receptor (CAR)-T cell, a CAR-NK cell, a CAR-macrophage cell, a Tumour-Infiltrating Lymphocyte (TIL) and a combination thereof.
115. Method of any one of embodiments 1 to 114, wherein the biopharmaceutical is an antibody.
116. Method of any one of embodiments 1 to 115, wherein the biopharmaceutical is an antibody-drug conjugate (ADC).
117. Method of any one of embodiments 1 to116, wherein the biopharmaceutical is an oligonucleotide.
118. Method of any one of embodiments 1 to 117, wherein the biopharmaceutical is an oncolytic virus.
119. Method of any one of embodiments 1 to 118, wherein the biopharmaceutical is a CAR-T cell.
120. Method of any one of embodiments 1 to 119, wherein the biopharmaceutical is a CAR-NK cell.
121. Method of any one of embodiments 1 to 120, wherein the biopharmaceutical is a CAR- macrophage cell.
122. Method of any one of embodiments 1 to 121, wherein the biopharmaceutical is a TIL.
123. Method of any one of embodiments 1 to 122, wherein the therapeutic agent is a chemotherapeutic.
124. Method of any one of embodiments 1 to 123, wherein the therapeutic agent is an immune therapeutic.
125. Method of any one of embodiments 1 to 124, wherein the tumour sample is a sample of a primary tumour.
126. Method of any one of embodiments 1 to 125, wherein the tumour sample is a sample of a metastasis.
127. Method of any one of embodiments 1 to 126, wherein the tumour sample is the sample of a metastasis where the primary tumour is known.
128. Method of any one of embodiments 1 to 126, wherein the tumour sample is the sample of a metastasis where the primary tumour is unknown.
129. Kit for testing the effect of an agent on a tumour cell according to a method of any one of embodiments 1 to 128, wherein the kit comprises a vessel, a tumour marker, a marker for an immune cell, and optionally a marker for dying and/or dead cells, a cytokine marker, and/or a marker for a therapeutic and/or diagnostic molecule.
130. Kit of embodiment 129, wherein the vessel is a dish such as a Petri dish.
131. Kit of embodiment 129, wherein the vessel is a well.
132. Kit of embodiment 19 or 131, wherein the vessel is a multi-well plate such as a 384 well plate.
133. Kit of any one of embodiments 129 to 132, wherein the agent is a therapeutic agent.
134. Kit of any one of embodiments 129 to 133, wherein the therapeutic agent is a small molecule, a biopharmaceutical, or a combination thereof.
135. Kit of any one of embodiments 129 to 134, wherein the biopharmaceutical is selected from the group consisting of an antibody, an antibody-drug conjugate (ADC), an oligonucleotide, an oncolytic virus, a Chimeric Antigen Receptor (CAR)-T cell, a CAR-NK cell, a CAR-macrophage cell, a Tumour-Infiltrating Lymphocyte (TIL) and a combination thereof.
136. Kit of any one of embodiments 129 to 135, wherein the biopharmaceutical is an antibody.
137. Kit of any one of embodiments 129 to 136, wherein the biopharmaceutical is an antibody-drug conjugate (ADC).
138. Kit of any one of embodiments 129 to 137, wherein the biopharmaceutical is an oligonucleotide.
139. Kit of any one of embodiments 129 to 138, wherein the biopharmaceutical is an oncolytic virus.
140. Kit of any one of embodiments 129 to 139, wherein the biopharmaceutical is a CAR-T cell.
141. Kit of any one of embodiments 129 to 140, wherein the biopharmaceutical is a CAR-NK cell.
142. Kit of any one of embodiments 129 to 141, wherein the biopharmaceutical is a CAR-macrophage cell.
143. Kit of any one of embodiments 129 to 142, wherein the biopharmaceutical is a TIL.
144. Kit of any one of embodiments 129 to 143, wherein the therapeutic agent is a chemotherapeutic.
145. Kit of any one of embodiments 129 to 144, wherein the therapeutic agent is an immune therapeutic.
146. Kit of any one of embodiments 129 to 145, wherein the marker for the cell nucleus is 4', 6-diamin-2-phenylindol (DAPI) or 2'-[4-ethoxyphenyl]-5-[4-methyl-1-piperazinyl]-2,5'-bi-1H-benzimidazole trihydrochloride trihydrate (Hoechst 33342). 147. Kit of any one of embodiments 129 to 146, wherein the tumour marker is selected from the group consisting of EpCAM, CK1, CK2, CK4, CK5, CK6, CK7, CK8, CK10, CK13, CK14, CK17, CK18, CK19, CK20, E-Cadherin, EMA, TTF-1, Napsin A, p63, p40, p53, GATA3, Mammaglobin, GCDFP-15, CDX2, SATB2, PAX8, Uroplakin II, Uroplakin III, PSA, NKX3.1, Prostein, Claudin-4, Ki-67, CD56, MCSP, gp100 and a combination thereof.
148. Method of any one of embodiments 1 to 147, wherein the tumour marker is selected from the group consisting of EpCAM, CK1, CK2, CK4, CK5, CK6, CK7, CK8, CK10, CK13, CK14, CK17, CK18, CK19, CK20 and a combination thereof.
149. Kit of any one of embodiments 129 to 148, wherein the tumour marker is selected from the group consisting of E-Cadherin, EMA, TTF-1, Napsin A, p63, p40, p53, GATA3, Mammaglobin, GCDFP-15, CDX2, SATB2, PAX8, Uroplakin II, Uroplakin III, PSA, NKX3.1, Prostein, Claudin-4, Ki-67, CD56, MCSP, gp100 and a combination thereof.
150. Kit of any one of embodiments 129 to 149, wherein the tumour marker is EpCAM.
151. Kit of any one of embodiments 129 to 150, wherein the tumour marker is CK1.
152. Kit of any one of embodiments 129 to 151, wherein the tumour marker is CK2.
153. Kit of any one of embodiments 129 to 152, wherein the tumour marker is CK4.
154. Kit of any one of embodiments 129 to 153, wherein the tumour marker is CK5.
155. Kit of any one of embodiments 129 to 154, wherein the tumour marker is CK6.
156. Kit of any one of embodiments 129 to 155, wherein the tumour marker is CK7.
157. Kit of any one of embodiments 129 to 156, wherein the tumour marker is CK8.
158. Kit of any one of embodiments 129 to 157, wherein the tumour marker is CK10.
159. Kit of any one of embodiments 129 to 158, wherein the tumour marker is CK13.
160. Kit of any one of embodiments 129 to 159, wherein the tumour marker is CK14.
161. Kit of any one of embodiments 129 to 160, wherein the tumour marker is CK17.
162. Kit of any one of embodiments 129 to 161, wherein the tumour marker is CK18.
163. Kit of any one of embodiments 129 to 162, wherein the tumour marker is CK19.
164. Kit of any one of embodiments 129 to 163, wherein the tumour marker is CK20.
165. Kit of any one of embodiments 129 to 164, wherein the marker for the immune cell is selected from the group consisting of CD45, CD3, CD4, CD8, CD19, CD20, CD45, CD14, CD16, CD56, CD25, CD127, CD11b, CD11c, CD15, CD33, CD34, CD38, CD68, CD45RA, CD45RO, HLA-DR, CD66b, CD27, CD28, CD62L, CCR7, CXCR3, CXCR5, FOXP3, CD68 and a combination thereof.
166. Kit of any one of embodiments 129 to 165, wherein the marker for the immune cell is selected from the group consisting of CD45, CD3, CD4, CD8 and a combination thereof.
167. Kit of any one of embodiments 129 to 165, wherein the marker for the immune cell is selected from the group consisting of CD19, CD20, CD45, CD14, CD16, CD56, CD25, CD127, CD11b, CD11c, CD15, CD33, CD34, CD38, CD68, CD45RA, CD45RO, HLA-DR, CD66b, CD27, CD28, CD62L, CCR7, CXCR3, CXCR5, FOXP3, CD68 and a combination thereof.
168. Kit of any one of embodiments 129 to 167, wherein the immune cell marker is CD45.
169. Kit of any one of embodiments 129 to 168, wherein the immune cell marker is CD3.
170. Kit of any one of embodiments 129 to 169, wherein the immune cell marker is CD4.
171. Kit of any one of embodiments 129 to 170, wherein the immune cell marker is CD8.
172. Kit of any one of embodiments 129 to 171, wherein the tumour marker is EpCAM and the immune cell marker is CD45.
173. Kit of any one of embodiments 129 to 172, wherein the tumour sample is a fresh tumour sample.
174. Kit of any one of embodiments 129 to 173, wherein the sample is a biopsy.
175. Kit of any one of embodiments 129 to 174, wherein the biopsy is a needle biopsy.
176. Kit of any one of embodiments 129 to 175, wherein the biopsy is a fine needle biopsy.
177. Kit of any one of embodiments 129 to 176, wherein the biopsy is a liquid biopsy.
178. Kit of any one of embodiments 129 to 177, wherein the liquid biopsy is a malignant effusion. 179. Kit of any one of embodiments 129 to 178, wherein the liquid biopsy is a pleural effusion.
180. Kit of any one of embodiments 129 to 179, wherein the liquid biopsy is pericardial effusion.
181. Kit of any one of embodiments 129 to 180, wherein the liquid biopsy is ascites. 182. Method of any one of embodiments 129 to 181, wherein the liquid biopsy is frozen.
183. Kit of any one of embodiments 129 to 182, wherein the single cell suspension has a concentration of 0.01 to 1×10⁶ cells/ml.
184. Kit of embodiment 183, wherein the single cell suspension has a concentration of 0.1 ×10⁶ cells/ml.
185. Kit of embodiment 183, wherein the single cell suspension has a concentration of 1 ×10⁶ cells/ml.
186. Kit of embodiment 183, wherein the single cell suspension has a concentration of 0.0125 or 0.016 ×10⁶ cells/ml.187. Kit of any one of embodiments 129 to 186, wherein the marker for cell viability is a cytokine marker.
188. Kit of any one of embodiments 129 to 187, wherein the cytokine marker is selected from the group consisting of IL-4, IL-2, IP-10, IL-1β, TNF-α, MCP-1, IL-17a, IL-6, IL-10, IFN-y, IL-12p70, IL-8, Free active TGF-β1, Fas, FasL, Granzyme A, Granzyme B, Perforin, Granulysin, Arginase, TARC, IL-1RA, IL-12p40, IL-23 and a combination thereof.
189. Kit of any one of embodiments 129 to 188, wherein the marker for a therapeutic and/or diagnostic molecule is selected from the group consisting of PD-L1, PD-1, CTLA-4, TIGIT, TIM-3, LAG-3, ICOS, VISTA, CD112R, BTLA, GITR, NKG2A, HER2 (ERBB2), EGFR, ALK, ROS1, MET, BRAF, KRAS, NRAS, PSMA, GPC3, CEA, CA19-9, CA125, MUC1, WT1, NY-ESO-1, mesothelin, VEGF, FGFR1, FGFR2, FGFR3, RET, NTRK1, NTRK2, NTRK3, PARP, BRCA1, BRCA2Nectin-4, TROP2, Mesothelin, GPNMB, CD70, CEACAM-5, LIV-1, HER3, c-Met. and a combination thereof.
190. Kit of any one of embodiments 129 to 189, wherein the kit comprises a vessel, a tumour marker, a marker for an immune cell, and/or a marker for dying and/or dead cells.
191. Kit of any one of embodiments 129 to 190, wherein the kit comprises a vessel, a tumour marker, a marker for an immune cell, a marker for dying and/or dead cells, and/or a cytokine marker.
192. Kit of any one of embodiments 129 to 191, wherein the kit comprises a vessel, a tumour marker, a marker for an immune cell, a marker for dying and/or dead cells, a cytokine marker, and/or a marker for a therapeutic and/or diagnostic molecule.

## Claims

1. Method for testing the effect of an agent on a tumour sample without cultivation or expansion of the tumour sample prior to the following steps of:
a) mechanical treatment and/or enzymatic digestion of the tumour sample to produce a single cell suspension,
b) determining viability of the cells of the single cell suspension and seeding the single cell suspension on a test vessel for 15 to 60 min at room temperature for cell settling in the test vessel,
c) adding the agent to the settling and/or settled cells and incubating the cells on the test vessel with the agent at a single concentration or varying concentrations at 25-40 °C for 1 to 10 days,
d) collecting a sample of the supernatant from the test vessel for determining cytokine expression using a cytokine marker,
e) staining dying and/or dead cells in the test vessel using a marker for dying and/or dead cells,
f) fixing cells in the test vessel for performing immune fluorescence to determine a tumour marker, a marker for an immune cell and a marker for the cell nucleus such as 4', 6-diamin-2-phenylindol (DAPI) or 2'-[4-ethoxyphenyl]-5-[4-methyl-1-piperazinyl]-2,5'-bi-1H-benzimidazole trihydrochloride trihydrate (Hoechst 33342),
g) determining the number of viable immune and/or tumour cells in the test vessel during and/or after incubation, and/or optionally determining a marker for a therapeutic target and/or diagnostic molecule of viable immune and/or tumour cells or all cells of the tumour sample in the test vessel during and/or after incubation,
h) calculating a tumour-specific cytotoxic effect of the agent based on the specific reduction of the number of viable tumour cells in relation to the number of viable immune cells and/or
i) determining an immune modulatory effect of the agent based on the interaction of immune cells with tumour cells and/or optionally based on the results of the cytokine analysis of step d).

2. Method of claim 1, wherein the tumour sample is a fresh tumour sample or a fresh biopsy such as a needle biopsy, a fine needle biospy or a liquid biopsy such as a pleural effusion, a pericardial effusion or ascites, or wherein the tumour sample is a frozen liquid biopsy.

3. Method of claims 1 or 2, wherein the single cell suspension is seeded in the test vessel at a cell density of 123.5 cells per mm² to 2469.1 cells per mm².

4. Method of any one of claims 1 to 3, wherein the marker for dying and/or dead cells is selected from the group consisting of an antibody, a fluorescent dye, a radioactive dye and a combination thereof.

5. Method of any one of claims 1 to 4, wherein the tumour marker is selected from the group consisting of EpCAM, CK1, CK2, CK4, CK5, CK6, CK7, CK8, CK10, CK13, CK14, CK17, CK18, CK19, CK20, E-Cadherin, EMA, TTF-1, Napsin A, p63, p40, p53, GATA3, Mammaglobin, GCDFP-15, CDX2, SATB2, PAX8, Uroplakin II, Uroplakin III, PSA, NKX3.1, Prostein, Claudin-4, Ki-67, CD56, MCSP, gp100 and a combination thereof.

6. Method of any one of claims 1 to 5, wherein the marker for the immune cell is selected from the group consisting of CD45, CD3, CD4, CD8, CD19, CD20, CD45, CD14, CD16, CD56, CD25, CD127, CD11b, CD11c, CD15, CD33, CD34, CD38, CD68, CD45RA, CD45RO, HLA-DR, CD66b, CD27, CD28, CD62L, CCR7, CXCR3, CXCR5, FOXP3, CD68 and a combination thereof.

7. Method of any one of claims 1 to 6, wherein the tumour marker is EpCAM and the immune cell marker is CD45.

8. Method of any one of claims 1 to 7, wherein the tumour sample is mechanically minced into pieces of 0.5 to 5 mm³ in a suspension medium, incubated for 10 to 60 min at 20 °C to 40 °C and resuspended every 10 min to form the single cell suspension in step a).

9. Method of any one of claims 1 to 8, wherein the tumour sample is a liquid biopsy which is centrifuged in step b), optionally red blood cells are lysed, and the centrifuged cells are resuspended as single cell suspension, for example wherein the cell concentration in the single cell suspension is 0.01 to 1×10⁶ cells/ml.

10. Method of any one of claims 1 to 9, wherein the tumour sample or the single cell suspension of the mechanical treatment is enzymatically digested.

11. Method of any one of claims 1 to 10, wherein dead cells are removed if the cell viability is below 70 % in step b).

12. Method of any one of claims 1 to 11, wherein a fluorescence signal of the marker is sequentially measured using fluorescence microscopy in channels wherein laser and bandpass filter sets are set to non-overlapping channels.

13. Method of any one of claims 1 to 12, wherein the agent is a therapeutic agent for example selected from the group consisting of a small molecule, a biopharmaceutical such as an antibody, an antibody-drug conjugate (ADC), an oligonucleotide, an oncolytic virus, a Chimeric Antigen Receptor (CAR)-T cell, a CAR-NK cell, a CAR-macrophage cell, a Tumour-Infiltrating Lymphocyte (TIL), and a combination thereof.

14. Method of any one of claims 1 to 13, wherein the tumour sample is a sample of a primary tumour or of a metastasis.

15. Kit for testing the effect of an agent on a tumour cell according to a method of any one of claims 1 to 14, wherein the kit comprises a vessel, a tumour marker, a marker for an immune cell, and optionally a marker for dying and/or dead cells, a marker for cytokines, and/or a marker for a therapeutic target and/or diagnostic molecule.
